# EUROPEAN PATENT SPECIFICATION

(11) **EP 1 038 015 B1**
(45) Date of publication and mention of the grant of the patent: **07.06.2006**
(21) Application number: 98966865.2
(22) Date of filing: 17.12.1998
(51) Int. Cl.: C12N 15/85, C07K 14/47, C07K 16/18, C12Q 1/68

(54) **MOOD DISORDER GENE**
GEN, DAS IM ZUSAMMENHANG STEHT MIT GEMÜTSKRANKHEITEN
GENE DES TROUBLES DE L'HUMEUR

(30) Priority: 18.12.1997 GB 9726804
(43) Date of publication of application: 27.09.2000
(60) Divisional application: 05012283.7
(73) Proprietor: Vlaams Interuniversitair Instituut voor Biotechnologie, 9052 Zwijnaarde (BE)
(72) Inventor: VAN BROECKHOVEN, Christine, B-2650 Edegem (BE); RAEYMAEKERS, Peter, B-2547 Lint (BE); DEL-FAVERO, Jurgen, B-3300 Tienen (BE)
(74) Representative: Baldock, Sharon Claire
(86) International application number: PCT/EP1998/008543
(87) International publication number: WO 1999/032643

(56) References cited:
- WO-A-97/11175
- WO-A-97/17445
- WO-A-97/37043
- EWALD H ET AL: "SUSCEPTIBILITY LOCI FOR BIPOLAR AFFECTIVE DISORDER ON CHROMOSOME 18? A REVIEW AND A STUDY OF DANISH FAMILIES" PSYCHIATRIC GENETICS, vol. 7, 1997, pages 1-12, XP002911589 ISSN: 0955-8829
- BRESCHEL T.S. ET AL.,: "A novel heritable expanding CTG repeat in an intron of the SEF2-1 gene on chromosome 18q21.1" HUMAN MOLECULAR GENETICS, vol. 6, no. 11, - 11 October 1997 (1997-10-11) pages 1855-1863, XP002112411
- TURECKI G ET AL: "eVIDENCEFOR A ROLE OF PHOSPHOLIPASE C-GAMMA.1 IN THE PATHOGENESIS OF BIPOLAR DISORDER" MOLECULAR PSYCHIATRY, vol. 3, no. 6, 1 January 1998 (1998-01-01), pages 534-538, XP002091617 ISSN: 1359-4184
- O'DONOVAN M.C. ET AL.,: "Expanded CAG repeats in schizophrenia and bipolar disorder" NATURE GENETICS, vol. 10, - August 1995 (1995-08) pages 380-381, XP002112412 cited in the application
- VERHEYEN G.R. ET AL.,: "Genetic refinement and physical mapping of a chromosome 18q candidate region for bipolar disorder" EUROPEAN JOURNAL OF HUMAN GENETICS, vol. 7, no. 4, - May 1999 (1999-05) pages 427-434, XP002112413
- GOOSSENS D. ET AL.: "No evidence for the involvement of CAG/CTG repeats from within 18q21.33-18q23 in bipolar disorder" EUROPEAN JOURNAL OF HUMAN GENETICS, MACMILLIAN PUBLISHERS LTD., vol. 8, 2000, pages 385-388,
- MCMAHON F. ET AL.: "Linkage of bipolar affective disorder to chromosome 18 markers in a new pedigree series" AMERICAN JOURNAL OF HUMAN GENETICS, vol. 61, 5 December 1997 (1997-12-05), pages 1397-1404, US

### SEQUENCE LISTING

<110> Vlaams Interuniversitair Instituut Voor Biotechnologie
<120> MOOD DISORDER GENE
<130> SCB/NLW/P48464/006
<140> EP 989 66 865.2
   <141> 1998-12-17
<150> GB 9726804.9
   <151> 1997-12-18
<150> PCT/EP9808543
   <151> 1998-12-17
<160> 162
<170> Patent In version 3.1
<210> 1
   <211> 167
   <212> DNA
   <213> Homo sapiens
<400> 1
   gtctttattt catataacta tgctctgatc tttgttactt tctcctttta actcagttta     60
   agctttattc ttattttcca gctgctgaag gtatatagtt aggttgttta ttggatacca 120
   ttctttcccg ttaatgtcag tggttactgc tatcaatgta gcagtta 167
<210> 2
   <211> 122
   <212> DNA
   <213> Homo sapiens
<400> 2
   ataaggtata ttatttgtgt cgtgagttaa gaaatcatta ataactattt tcagaatgac 60
   aaatgtcatt atatgttgta aaaaagataa atacgtgaaa ttatgaggtt aagaaaagtt 120
   ta 122
<210> 3
   <211> 154
   <212> DNA
   <213> Homo sapiens
<220>
   <221> misc_feature
   <222> (109) .. (109)
   <223> n = a, c, g, or t/u
<220>
   <221> misc_feature
   <222> (134)..(134)
   <223> n = a, c, g, or t/u
<400> 3
   acataaaatg tcgctcaaaa acaattatgt gtgtctacac atatgggaaa gcaggaaaca 60
   aatttgttta caacatacat tacttttgtt ttttaggcaa gataaaatnt cctacctcca 120
   aaaccaccag cacngtccgc aataactata catc 154
<210> 4
   <211> 301
   <212> DNA
   <213> Homo sapiens
<220>
   <221> misc_feature
   <222> (217) .. (217)
   <223> n = a, c, g, or t/u
<220>
   <221> misc_feature
   <222> (298)..(298)
   <223> n = a, c, g, or t/u
<400> 4
   aatatcattc ttcacccacg ttatacataa gagaccagaa tgtgatattg tcatctcaca 60
   tggaaaaatc tgctgtgatc agttcctgaa gcttgctgtg atcctccctt aggaaagtag 120
   aaaaatcttt ttgaaacact ttattctaca atcaatgaaa attaggtgaa gctacagaag 180
   ccagaaatta ctctaagatt agacaattat ttaagangac caattgtctt tggtcttctt 240
   ctgaagggtc tgactaccct cctccaaaga attcactggc cgtcgtttta caacgtcntg 300
   a 301
<210> 5
   <211> 191
   <212> DNA
   <213> Homo sapiens
<220>
   <221> misc_feature
   <222> (11)..(11)
   <223> n = a, c, g, or t/u
<220>
   <221> misc_feature
   <222> (17)..(17)
   <223> n = a, c, g, or t/u
<220>
   <221> misc_feature
   <222> (62)..(62)
   <223> n = a, c, g, or t/u
<220>
   <221> misc_feature
   <222> (162)..(162)
   <223> n = a, c, g, or t/u
<400> 5
   ggagggtgtt ntcacanaag tctggggtgc gctgtgttgt tcattgtaaa aaccctttgg 60
   ancatctggg aatgtgctgc cccacatgtc caggtaacgt tctcaggaag gggaggctgg 120
   aaatctctgt gtgttcttac aggaatgcat gaaatctccc ancccctctt gttggaaatt 180
   tccctcactt t 191
<210> 6
   <211> 253
   <212> DNA
   <213> Homo sapiens
<220>
   <221> misc_feature
   <222> (7) .. (7)
   <223> n = a, c, g, or t/u
<220>
   <221> misc_feature
   <222> (250)..(250)
   <223> n = a, c, g, or t/u
<220>
   <221> misc_feature
   <222> (12)..(12)
   <223> n = a, c, g, or t/u
<220>
   <221> misc_feature
   <222> (217)..(217)
   <223> n = a, c, g, or t/u
<400> 6
   cttctcnatg antggacaaa tgtcattggg tcagcatgag gcacagctta ccagttcaga     60
   ttccagtagc tgaggaacaa atcttaactc caaaaataag taattgcgtc actttggagg    120
   aattatttga ccttttcata actttgacat cacaacaatg agggtgaagt tagtaaaata    180
   aatgattatt atgaggataa aatgagaaaa tgaattnagt gcttaagaca atgcttggta    240
   actagttaan ccg                                                       253
<210> 7
   <211> 153
   <212> DNA
   <213> Homo sapiens
<220>
   <221> misc_feature
   <222> (4) .. (4)
   <223> n = a, c, g, or t/u
<400> 7
   ggtntttcac ttggttggtt aacattactt ctaagttttt tattgttttt tatgctattg     60
   ctaatgggat tgctttctta atttattttt tccaatagct tgttgttagt ttatatcaaa    120
   tgcaactgtt tttctatgca aattatgttt cct                                 153
<210> 8
   <211> 238
   <212> DNA
   <213> Homo sapiens
<220>
   <221> misc_feature
   <222> (130)..(130)
   <223> n = a, c, g, or t/u
<220>
   <221> misc_feature
   <222> (141) .. (141)
   <223> n = a, c, g, or t/u
<220>
   <221> misc_feature
   <222> (176)..(176)
   <223> n = a, c, g, or t/u
<220>
   <221> misc_feature
   <222> (235) .. (235)
   <223> n = a, c, g, or t/u
<400> 8
   ttggtggtgc cctaggtttg gcaattataa ataaagctgc tacaaacatt catgtgcagg     60
   tctccgtgtg gacataattt tccagttcat ttgggtaaaa cccaagggag cacaactgtt    120
   ggatcctatn ataaaaatat ntctcgtttc atttaaaaaa cctgggaaac tatctnccca    180
   cagtggctgt ccctttttgt atccccacca acaatgttgg aaagcctatt gccancat       238
<210> 9
   <211> 182
   <212> DNA
   <213> Homo sapiens
<220>
   <221> misc_feature
   <222> (5) .. (5)
   <223> n = a, c, g, or t/u
<220>
   <221> misc_feature
   <222> (72) .. (72)
   <223> n = a, c, g, or t/u
<220>
   <221> misc_feature
   <222> (86)..(86)
   <223> n = a, c, g, or t/u
<220>
   <221> misc_feature
   <222> (106)..(106)
   <223> n = a, c, g, or t/u
<220>
   <221> misc_feature
   <222> (130)..(130)
   <223> n = a, c, g, or t/u
<220>
   <221> misc_feature
   <222> (145)..(145)
   <223> n = a, c, g, or t/u
<400> 9
   catgnctcac agtgttctga ggctgctctg gacatgcaat cttgcatgct tttgtcatga     60
   caggtcttaa anagtttatc agcttnctca aatagctgaa tgacanaaca ctggattttt    120
   gttcaaatan cctatcaact tggcntctgt gttgcggttg tcacttggta acaaaataag    180
   tc                                                                   182
<210> 10
   <211> 259
   <212> DNA
   <213> Homo sapiens
<220>
   <221> misc_feature
   <222> (29) .. (29)
   <223> n = a, c, g, or t/u
<220>
   <221> misc_feature
   <222> (122)..(123)
   <223> n = a, c, g, or t/u
<220>
   <221> misc_feature
   <222> (146)..(146)
   <223> n = a, c, g, or t/u
<220>
   <221> misc_feature
   <222> (192)..(193)
   <223> n = a, c, g, or t/u
<220>
   <221> misc_feature
   <222> (213)..(213)
   <223> n = a, c, g, or t/u
<400> 10
   taattgacaa ataaaaattg tatattttnc atatttaaca tgttatgcta acatatatat 60
   ggattgtgga atggctaagt cagaaattct tttacattca tatttccata ttatttactt 120
   tnngctttaa aaaatatgta aatganaata cttatttttt tcagtgtcac tgccttgata 180
   ctttcacatt tnngttacat attatttccc ttncatctaa caaatatata ttgagtttct 240
   ataatgtgtc tgacactga 259
<210> 11
   <211> 195
   <212> DNA
   <213> Homo sapiens
<220>
   <221> misc_feature
   <222> (90)..(90)
   <223> n = a, c, g, or t/u
<400> 11
   tggtcactgg tgccttattt ggtttgtttg ctgaggtcat atttcctgtg gccttcatgc 60
   ttgatttgtt ggagtctagc catgtaaaan tctgttggag tctaggcatt taaaaaatag 120
   gtatttattg taatctttgc catttgcttg tttgtatcca tccttcttgg gaaggcttta 180
   caggcattca aaagg 195
<210> 12
   <211> 656
   <212> DNA
   <213> Homo sapiens
<220>
   <221> misc_feature
   <222> (556)..(556)
   <223> n = a, c, g, or t/u
<220>
   <221> misc_feature
   <222> (590)..(590)
   <223> n = a, c, g, or t/u
<220>
   <221> misc_feature
   <222> (610)..(610)
   <223> n = a, c, g, or t/u
<400> 12
   gccaacaaac aaaatgaaat aagacctggg atgtattttt tggccaaggc aattagaaaa    60
   tgattagtat ccttatcagg agcaatttca gagaatgttt gggtggacgt ctaactacag   120
   tggagtcaaa cgtgaatcaa cggtgaaaaa aggacaatag ccaatgtgta cactttttat   180
   aaaaaccacc ctccaaggac caggcactgg ccctctctcc ggtgcccaca gacatccaca   240
   caggcccaaa gaatcaggga ttgcacaagc cagagcaatc gaacggttct gagtcatctg   300
   ccggaagcct tgccctcaat caaggcggac gtgaagcatc tacaaaggag gaatagtcaa   360
   agcagcagcg gcggcggcgg cggcggcagc agcagcagca gcaggaggtg ggggcctctg   420
   ccaggtaccg ggcggggcag gcacggaggt gcccaggttc ccgcggaggc cacctcttcc   480
   ctggagtgcg tgagagaggg gaagggagga aggccagagc aggaatcaga gcgaggcaaa   540
   ggcgggcagg aactangaga atgacsgcgg gaggcggccg ggaaagaaan tctcggggct   600
   gtgggggtcn ccctggcacc agccggggtc ccaagcccca ccgcgagacc ccgcga        656
<210> 13
   <211> 22
   <212> DNA
   <213> Homo sapiens
<400> 13
   atcgaacggt tctgagtcat ct 22
<210> 14
   <211> 19
   <212> DNA
   <213> Homo sapiens
<400> 14
   cgctctgatt cctgctctg 19
<210> 15
   <211> 546
   <212> DNA
   <213> Homo sapiens
<400> 15
   ttcagtagaa ggaagcacag caaatttgcc tttatagaga ttcaattctt ggtgcttggg      60
   ccaaagaata agaattacat taagcaggcc gggcacggtg gctcacacct gtaaaaccag     120
   aactttggga ggccgaggca ggcagatcat gaggtcagga gatcgagacc atcctggaca     180
   acatagtgaa accccatctc tactaaaaat acaaaaatta gccgggcatg gtggtgcatg     240
   cctgtaatcc cagctactca ggaggcggag gcaggagaat cccttgaacc agggagttgg     300
   aggttgcagt gagccgagat cacgccacag cactctagcc tggcgacaga gtgagactcc     360
   atctcaaaaa aaaaaaaaaa aaaaaaaaaa ttacattaag cagcagcagc agcagtgasa     420
   gagggaakaa tgaaagaaga aatttctaga ataagattga tctccagcac catgccaatc     480
   atggactgga tacaattcat gcatatcttt tgtgagagag gtgagagatg tgaatccttt     540
   ctcatt                                                                546
<210> 16
   <211> 22
   <212> DNA
   <213> Homo sapiens
<400> 16
   agaaggaagc acagcaaatt tg 22
<210> 17
   <211> 20
   <212> DNA
   <213> Homo sapiens
<400> 17
   gcatggtgct ggagatcaat 20
<210> 18
   <211> 573
   <212> DNA
   <213> Homo sapiens
<220>
   <221> misc_feature
   <222> (28) .. (28)
   <223> n = a, c, g, or t/u
<220>
   <221> misc_feature
   <222> (74)..(74)
   <223> n = a, c, g, or t/u
<220>
   <221> misc_feature
   <222> (92)..(92)
   <223> n = a, c, g, or t/u
<220>
   <221> misc_feature
   <222> (97) .. (97)
   <223> n = a, c, g, or t/u
<220>
   <221> misc_feature
   <222> (100)..(100)
   <223> n = a, c, g, or t/u
<220>
   <221> misc_feature
   <222> (123)..(123)
   <223> n = a, c, g, or t/u
<400> 18
   tgggagttaa agcagacatt cggctttngt gttgccagag ttctaacata agttcttttt     60
   catctgggca ggcngatgtt ccttccatct tngaagnacn gtccttttca ttttttttat    120
   ttngcttttg gsktttatct tcttagacgt cttcaggagt tkgattgtag kgtaaggcag   180
   atttagttga ctgggctttg tttctggaaa attttaaagg ggcaagtcct gggctgcata    240
   ttcttactct gggggcttag tactggcccc taaatttgtt ctctggctcc tcaaggttag    300
   aaatctgctg gctggagggg ctgagatgtt ccttgactgc tggccagaac attccgccgg    360
   ggggtggcaa ccgaagtgtt tctttgggca atggcagcag aattcatgat tgttttcatg    420
   trccagcagc agtggcagcg caktgagttg catgattgtt ggctggggct gagtgctggc    480
   asgcactgga gtgtttggct tccagtagaa attcacagca gtagtagtgg tggcatggga    540
   aggagggcag yggtggcatg gggaggaccc ccc                                 573
<210> 19
   <211> 22
   <212> DNA
   <213> Homo sapiens
<400> 19
   ggctgagatg ttccttgact gc 22
<210> 20
   <211> 22
   <212> DNA
   <213> Homo sapiens
<400> 20
   ccttcccatg ccaccactac ta 22
<210> 21
   <211> 597
   <212> DNA
   <213> Homo sapiens
<220>
   <221> misc_feature
   <222> (67) .. (67)
   <223> n = a, c, g, or t/u
<220>
   <221> misc-feature
   <222> (95)..(95)
   <223> n = a, c, g, or t/u
<220>
   <221> misc_feature
   <222> (151)..(151)
   <223> n = a, c, g, or t/u
<400> 21
   tgtaattccc agcaatttgg ggagcccaag gcgggcagat tcatgagttc gggaagattc 60
   gagaccnttc ctggctaaac acgggggaaa ccccnttttt actaaaaaat accaaaaaat 120
   taacctgggc gtggtggcgg gccccagcta ntccggaggc tgaggcagga gaatggtgtg 180
   aacccgggag gcggagcttg cagtgagccg agatcccgct actgcactcc agcctgggca 240
   atagagggag actccgtctc aaaaaaaaaa aaaaataaat aataataaaa aaaataacaa 300
   taataatact aataattgct tgatatttta caaaagcaaa aggaaaagaa gactaggcaa 360
   gaaaaaaaaa acctccttag atggtagaac tcaggtttaa aattaaaact tattctggtg 420
   tcagsctagt tgtatatttt gacctcttta aatgctctga actatgatat ggagtaacag 480
   cgatgctgct gctgctgctg ctgctgctga tggtggtggt gttttaatat cgaataaaag 540
   ttgtggaaac taaatttcat ttctgccaat taactaagat tgcaaagtta aacatct 597
<210> 22
   <211> 22
   <212> DNA
   <213> Homo sapiens
<400> 22
   tttgcaatct tagttaattg gc 22
<210> 23
   <211> 24
   <212> DNA
   <213> Homo sapiens
<400> 23
   gaactatgat atggagtaac agcg 24
<210> 24
   <211> 16
   <212> DNA
   <213> Homo sapiens
<400> 24
   cctggctcac ctggca 16
<210> 25
   <211> 23
   <212> DNA
   <213> Homo sapiens
<400> 25
   ttgtagcatc gttgtaatgt tcc 23
<210> 26
   <211> 261
   <212> DNA
   <213> Homo sapiens
<220>
   <221> misc_feature
   <222> (76)..(76)
   <223> n = a, c, g, or t/u
<220>
   <221> misc_feature
   <222> (80)..(80)
   <223> n = a, c, g, or t/u
<220>
   <221> misc_feature
   <222> (82)..(82)
   <223> n = a, c, g, or t/u
<220>
   <221> misc_feature
   <222> (167)..(167)
   <223> n = a, c, g, or t/u
<400> 26
   agctatcctg gctcacctgg caaaaataca gtgtatacac acacacacac acacacacac 60
   acacacacag agtgtnttan tnattccagc aaataatatt acatataaaa gatctaattg 120
   gttcatcatg taaatttagt aggaacatta caacgatgct acaaganttt atccaaaact 180
   gagatttcct tagaatatct gttaaaagta attttattca gttaatagaa attctattga 240
   aaacatcaaa cttataaagc t 261
<210> 27
   <211> 21
   <212> DNA
   <213> Homo sapiens
<400> 27
   gatcccataa agctacgagg g 21
<210> 28
   <211> 25
   <212> DNA
   <213> Homo sapiens
<400> 28
   gagtctaaag acaagaaagc attgc 25
<210> 29
   <211> 1166
   <212> DNA
   <213> Homo sapiens
<220>
   <221> misc_feature
   <222> (437)..(499)
   <223> n = a, c, g, or t/u
<220>
   <221> misc_feature
   <222> (942)..(966)
   <223> n = a, c, g, or t/u
<400> 29
   tcttcttacc ccttggaaga agactgtttc caaataattt gaacagcttg ctgctaaatg      60
   ggacccaatt tttggcctat agacacttat gtattgtttt cgaatacgtc agattggacc     120
   agtgctcttc aggaatgtgg ctgcaagcaa ggggctagaa gttcacctcc tgacagtatt     180
   attaatacta tgcaaatatg gaataggaga ccatttgatt ttctaggctt tgtggtagag     240
   aggtgaaggt atgagaatta atagcgtgtg aacaaagtaa agaacaggat tccagaatga     300
   tcattaaatt tgtttctatt tattcttttt tgccccccta gagattaagt ccagaaatgt     360
   actttctggc acataaagaa atcttgagga ctttgtttaa accttccata aaaaaacaat     420
   tttcggtttc tcgggtnnnn nnnnnnnnnn nnnnnnnnnn nnnnnnnnnn nnnnnnnnnn     480
   nnnnnnnnnn nnnnnnnnnt tctttctttg tgtattttat tcaagatgag ttggacccat     540
   tgccagtgag tctgaatgtc actgacagcc ctgtgttgtg ctcaggactc actctgctgc     600
   tggtggaaac tcatggcttc tctctctctt tgatcccata aagctacgag ggggacggga     660
   gagggcagtg caatgggaag taaagagata ttttccagta ggaaaagcaa tgctttcttg     720
   tctttagact caaatgctta gggaacgttt catttctcat tcatggggaa aggcagcctc     780
   cttaaatgtt ttctgaagag cggtaaaatc tagaagctta agaatttaca gttccttcaa     840
   taaccatgat gacctgaagt tcacctatcc cattttagca tctacttgtt tttcccatct     900
   cttcctttcc aattttgctt atactgctgt aatatttttg tnnnnnnnnn nnnnnnnnnn      960
   nnnnnngacc agctaaaatt ttcgacttga ctttttaact taactcatga attaattaaa    1020
   gcaaatgaaa aaattaaaaa gtgtgacttt ttctcggagc atatatgtag cttttaggaa    1080
   aggctgatga tggtataaag tttgctcatt aagaaaaaaa gacaaggctg attttgaaga    1140
   gagttgcttt tgaaataaaa tgatca                                         1166
<210> 30
   <211> 20
   <212> DNA
   <213> Homo sapiens
<400> 30
   agacattctc gcttccctga 20
<210> 31
   <211> 20
   <212> DNA
   <213> Homo sapiens
<400> 31
   aattttgacc ccttatgggc 20
<210> 32
   <211> 1366
   <212> DNA
   <213> Homo sapiens
<220>
   <221> misc_feature
   <222> (165)..(175)
   <223> n = a, c, g, or t/u
<400> 32
   taagtggcat agcccatgtt aagtcctccc tgacttttct gtggatgccg atttctgtaa     60
   actctgcatc cagagattca ttttctagat acaataaatt gctaatgttg ctggatcagg    120
   aagccgccag tacttgtcat atgtagcctt cacacagata gaccnnnnnn nnnnnccaat    180
   tctatctttt gtttcctttt ttcccataag acaatgacat acgcttttaa tgaaaaggaa    240
   tcacgttaga ggaaaaatat ttattcatta tttgtcaaat tgtccggggt agttggcaga    300
   aatacagtct tccacaaaga aaattcctat aaggaagatt tggaagctct tcttcccagc    360
   actatgcttt ccttctttgg gatagagaat gttccagaca ttctcgcttc cctgaaagac    420
   tgaagaaagt gtagtgcatg ggacccacga aactgccctg gctccagtga aacttgggca    480
   catgctcagg ctactatagg tccagaagtc cttatgttaa gccctggcag gcaggtgttt    540
   attaaaattc tgaattttgg ggattttcaa aagataatat tttacataca ctgtatgtta    600
   tagaacttca tggatcagat ctggggcagc aacctataaa tcaacacctt aatatgctgc    660
   aacaaaatgt agaatattca gacaaaatgg atacataaag actaagtagc ccataagggg    720
   tcaaaatttg ctgccaaatg cgtatgccac caacttacaa aaacacttcg ttcgcagagc    780
   ttttcagatt gtggaatgtt ggataaggaa ttatagacct ctagtagctg aaatgcaaga    840
   ccccaagagg aagttcagat cttaatataa attcactttc atttttgata gctgtcccat    900
   ctggtcatgt ggttggcact agactggtgg caggggcttc tagctgactc gcacagggat    960
   tctcacaata gccgatatca gaatttgtgt tgaaggaact tgtctcttca tctaatatga   1020
   tagcgggaaa aggagaggaa actactgcct ttagaaaata taagtaaagt gattaaagtg   1080
   ctcacgttac cttgacacat agtttttcag tctatgggtt tagttacttt agatggcaag   1140
   catgtaactt atattaatag taatttgtaa agttgggtgg ataagctatc cctgttgccg   1200
   gttcatggat tacttctcta taaaaaatat atatttacca aaaaattttg tgacattcct   1260
   tctcccatct cttccttgac atgcattgta aataggttct tcttgttctg agattcaata   1320
   ttgaatttct cctatgctat tgacaataaa atattattga actacc                   1366
<210> 33
   <211> 25
   <212> DNA
   <213> Homo sapiens
<400> 33
   gaaatgcaca taacatatat ttgcc 25
<210> 34
   <211> 24
   <212> DNA
   <213> Homo sapiens
<400> 34
   tgctcactgc ctatttaatg tagc 24
<210> 35
   <211> 316
   <212> DNA
   <213> Homo sapiens
<220>
   <221> misc_feature
   <222> (11) .. (11)
   <223> n = a, c, g, or t/u
<220>
   <221> misc_feature
   <222> (130)..(130)
   <223> n = a, c, g, or t/u
<220>
   <221> misc_feature
   <222> (219)..(219)
   <223> n = a, c, g, or t/u
<220>
   <221> misc_feature
   <222> (267)..(267)
   <223> n = a, c, g, or t/u
<220>
   <221> misc_feature
   <222> (278)..(278)
   <223> n = a, c, g, or t/u
<220>
   <221> misc_feature
   <222> (295)..(295)
   <223> n = a, c, g, or t/u
<220>
   <221> misc_feature
   <222> (298)..(298)
   <223> n = a, c, g, or t/u
<400> 35
   gttgtttgga ngcaggttta tttattatat acttgcaatt gaatataaga tacagacata     60
   tatatgtgtt atgtatttct agaaatgcac ataacatata tttgcctatt gtttaatgtt    120
   ttttccagan atttattaca gaagggcatg gagggatacc tacttattct tcattatgag    180
   aacaattaaa ggcatttatt agataggaaa ttaacaganc atctgcttct ataactttat    240
   tagctacatt aaataggcag tgagcantaa tttaaaanct caccattata taaantanta    300
   aataccaaag taaaag                                                    316
<210> 36
   <211> 22
   <212 > DNA
   <213> Homo sapiens
<400> 36
   tgctcttctg aacaacttct gc 22
<210> 37
   <211> 20
   <212> DNA
   <213> Homo sapiens
<400> 37
   atagaagggc atggagggat 20
<210> 38
   <211> 601
   <212> DNA
   <213> Homo sapiens
<220>
   <221> misc_feature
   <222> (581)..(594)
   <223> n = a, c, g, or t/u
<400> 38
   aactaagcgt gctgcttctg caaaagattt ttgtagatga gctgtgtgcc tcagaattgc 60
   tatttcaaat tgccaaaaat ttagagatgt tttctacata tttctgctct tctgaacaac 120
   ttctgctacc cactaaataa aaacacagaa ataattagac aattgtctat tataacatga 180
   caaccctatt aatcatttgg tcttctaaaa tgggatcatg cccatttaga ttttccttac 240
   tatcagttta tttttataac attaactttt actttgttat ttattatttt atataatggt 300
   gagttttaaa ttattgctca ctgcctattt aatgtagcta ataaagttat agaagcagat 360
   gatctgttaa tttcctatct aataaatgcc tttaattgtt ctcataatga agaataagta 420
   ggtatccctc catgcccttc tataataaat atctggaaaa aacattaaac aataggcaaa 480
   tatatgttat gtgcatttct agaaatacat aacacatata tatgtctgta tcttatattc 540
   aattgcaagt atataataaa taaacctgct tccaaacaac nnnnnnnnnn nnnnggaatt 600
   c 601
<210> 39
   <211> 20
   <212> DNA
   <213> Homo sapiens
<400> 39
   atgggagacg taatacaccc 20
<210> 40
   <211> 17
   <212> DNA
   <213> Homo sapiens
<400> 40
   atgctgctgg tctgagg 17
<210> 41
   <211> 371
   <212> DNA
   <213> Homo sapiens
<220>
   <221> misc_feature
   <222> (78) .. (78)
   <223> n = a, c, g, or t/u
<220>
   <221> misc_feature
   <222> (303)..(303)
   <223> n = a, c, g, or t/u
<220>
   <221> misc_feature
   <222> (313) .. (313)
   <223> n = a, c, g, or t/u
<220>
   <221> misc_feature
   <222> (324)..(324)
   <223> n = a, c, g, or t/u
<220>
   <221> misc_feature
   <222> (347)..(347)
   <223> n = a, c, g, or t/u
<400> 41
   aaagagttgg ggttgtgaat tcccacacca gtcaactatt ggctatgggc ttaccatggg      60
   agacgtaata cacccggnac ttccaactca cataccagag acatggctct agcacccaat     120
   ggaaatatgc tgaatgttgc aggtgcaaga cagcaacaaa gcagacagag gcacatagac     180
   aaggcaccaa cagtgtccac tataccctga cagtgtggaa agttgtagat aggatgaaga     240
   gaaagaatac acacacacac acacacacac acacacacac acacacacac acacacggta     300
   ganacttact acncaaagtg tgancctcag accagcagca tctggcnaaa tggtgatcta     360
   tcaccttcca g                                                          371
<210> 42
   <211> 24
   <212> DNA
   <213> Homo sapiens
<400> 42
   tgtgctactg attaaggtaa aggc 24
<210> 43
   <211> 24
   <212> DNA
   <213> Homo sapiens
<400> 43
   tgcttcttca atttgtagag ttgg 24
<210> 44
   <211> 224
   <212> DNA
   <213> Homo sapiens
<220>
   <221> misc_feature
   <222> (96)..(96)
   <223> n = a, c, g, or t/u
<220>
   <221> misc_feature
   <222> (129)..(129)
   <223> n = a, c, g, or t/u
<400> 44
   ctgagacaag gcaggcaaac aacctctaaa aatctacaat tggtgattgg tgtgctactg 60
   attaaggtaa aggcacagaa ttatacatcc aggttnctat tacttatggc agactcagga 120
   cccaggttna gagaccactg gccttaagaa aaaaaatggg gttcctgatt tctggataat 180
   aatccaactc tacaaattga agaagcaaca taccctcttt gtta 224
<210> 45
   <211> 23
   <212> DNA
   <213> Homo sapiens
<400> 45
   cttaatgaaa acaatgccag agc 23
<210> 46
   <211> 22
   <212> DNA
   <213> Homo sapiens
<400> 46
   tgcaaaatgt ggaataatct gg 22
<210> 47
   <211> 213
   <212> DNA
   <213> Homo sapiens
<400> 47
   ctacaaaatg catgtggctt tggctttgaa atagtacacc ctatcaaaga ctaaattttc 60
   ttaatgaaaa caatgccaga gcttttttca tgatattttg tttttagaga tggggaacaa 120
   tctggacgtt gtttccttat ctgggtggta atcgaggctt agcaatttcc cacagcgtta 180
   cacaaatcca gattattcca cattttgcaa ata 213
<210> 48
   <211> 20
   <212> DNA
   <213> Homo sapiens
<400> 48
   gggaagtcaa atgcaaaatc 20
<210> 49
   <211> 25
   <212> DNA
   <213> Homo sapiens
<400> 49
   agcttctgag taatcttatg ctgtg 25
<210> 50
   <211> 351
   <212> DNA
   <213> Homo sapiens
<220>
   <221> misc_feature
   <222> (32)..(32)
   <223> n = a, c, g, or t/u
<220>
   <221> misc_feature
   <222> (167)..(167)
   <223> n = a, c, g, or t/u
<400> 50
   agctgaacat gccttttcat ggagcagttt cnaaatacac ttttggtaca atctgcaggt     60
   ggatatttgg agctcaggag tttgagacca gcctgggcaa catggtgaaa tcccgtctct    120
   actaaaatac aaaaaattag ccaggtgtgg cggcatgtgc ctgtagnccc aggatggatt    180
   gagtgggtga gatatggaat aagtggtggg aagtcaaatg caaaatcaat tcagtttgtc    240
   aatattgatt ctctattcta gcctggcgtg gtttttcctc gtcacacaca cacacacaca    300
   cacacacaca cacacacaca cacacacaca gcataagatt actcagaagc t             351
<210> 51
   <211> 20
   <212> DNA
   <213> Homo sapiens
<400> 51
   aacaagtgtg tatgggggtg 20
<210> 52
   <211> 21
   <212> DNA
   <213> Homo sapiens
<400> 52
   catattcacc cagtttgttg c 21
<210> 53
   <211> 647
   <212> DNA
   <213> Homo sapiens
<220>
   <221> misc_feature
   <222> (113)..(113)
   <223> n = a, c, g, or t/u
<220>
   <221> misc_feature
   <222> (147)..(147)
   <223> n = a, c, g, or t/u
<220>
   <221> misc_feature
   <222> (185)..(185)
   <223> n = a, c, g, or t/u
<220>
   <221> misc_feature
   <222> (196)..(196)
   <223> n = a, c, g, or t/u
<220>
   <221> misc_feature
   <222> (207)..(207)
   <223> n = a, c, g, or t/u
<220>
   <221> misc_feature
   <222> (216).. (216)
   <223> n = a, c, g, or t/u
<220>
   <221> misc_feature
   <222> (221)..(221)
   <223> n = a, c, g, or t/u
<220>
   <221> misc_feature
   <222> (226) .. (226)
   <223> n = a, c, g, or t/u
<220>
   <221> misc_feature
   <222> (228) .. (228)
   <223> n = a, c, g, or t/u
<220>
   <221> misc_feature
   <222> (233)..(233)
   <223> n = a, c, g, or t/u
<220>
   <221> misc_feature
   <222> (237)..(237)
   <223> n = a, c, g, or t/u
<220>
   <221> misc_feature
   <222> (239)..(239)
   <223> n = a, c, g, or t/u
<220>
   <221> misc_feature
   <222> (376)..(376)
   <223> n = a, c, g, or t/u
<220>
   <221> misc_feature
   <222> (397)..(398)
   <223> n = a, c, g, or t/u
<400> 53
   cagggaaatg caaatcaaaa ccacaatgag ttatctcctc atacctttaa tgatggctaa      60
   tattaaacaa gagataacaa gtgtgtatgg gggtgtggag aaaagagaat gtncgaacac     120
   tcttggttga aatataagtt ggtagancca ttatgcaaaa cagtatgaat ctttatcagt     180
   ataanattag gacctngcat atgatcncag caatcnccac ntctgngnga tcncacncnc     240
   tatctctcta tatctatcta tctatctatc tatctatcta tctatctatc tatctatcta     300
   tctatctgtc tgtctatcat ctatctatct tctatctatc tatctatctt tctatctatc     360
   tatctgtcta tctatnccgg aatatttttc agccatnnaa ataaggaagt cctgctattt     420
   gcaacaaact gggtgaatat ggagaacgtt atgctaaatg caatatgcta aagacagaca     480
   cagaaagaca agtatgacct cacttatatg tggaaactga aaaagccata ctcattacag     540
   caaagagtag aatgttggtt accaggggca aagagggtag aaatgagggg agtgagaaaa     600
   tgtcaatcaa agtgtaagaa tgttataaca taaataaatt catagag                   647
<210> 54
   <211> 21
   <212> DNA
   <213> Homo sapiens
<400> 54
   gttgactcaa gtccaaacct g 21
<210> 55
   <211> 24
   <212> DNA
   <213> Homo sapiens
<400> 55
   caaagacatt gtagacgttc tctg 24
<210> 56
   <211> 336
   <212> DNA
   <213> Homo sapiens
<220>
   <221> misc_feature
   <222> (167)..(167)
   <223> n = a, c, g, or t/u
<400> 56
   agctgcatat aaaactattc catttcacat ttttgaagac atttgtagcc atgatacttt 60
   gctgttgtct gtgggccacc tctttttgaa gtgtgtagtt aactgtgctc ctgtaatctg 120
   ttgtctgttg actcaagtcc aaacctgttc tgcgtggcat gtttctncaa cttgatgtga 180
   tgctatttat cactttcttt gaagttaagt ctctatgtct ttgtattctt tctgtgtacc 240
   cagggatatg tttgtgcatg cacacgcata aacacacaca cacacacaca cacacacaga 300
   gacagagaca gagaacgtct acaatgtctt tgtgag 336
<210> 57
   <211> 25
   <212> DNA
   <213> Homo sapiens
<400> 57
   agccaatacc ttgtagtaaa tatcc 25
<210> 58
   <211> 22
   <212> DNA
   <213> Homo sapiens
<400> 58
   gattctccag acaaataatc cc 22
<210> 59
   <211> 350
   <212> DNA
   <213> Homo sapiens
<220>
   <221> misc_feature
   <222> (170)..(170)
   <223> n = a, c, g, or t/u
<220>
   <221> misc_feature
   <222> (288)..(288)
   <223> n = a, c, g, or t/u
<220>
   <221> misc_feature
   <222> (302)..(302)
   <223> n = a, c, g, or t/u
<220>
   <221> misc_feature
   <222> (310)..(310)
   <223> n = a, c, g, or t/u
<220>
   <221> misc_feature
   <222> (324)..(324)
   <223> n = a, c, g, or t/u
<220>
   <221> misc_feature
   <222> (333)..(333)
   <223> n = a, c, g, or t/u
<400> 59
   gagtgagcca ataccttgta gtaaatatcc atctatcttt gatgtatcta tgtatctatc 60
   tttgtatcta tatgtctatg tatctatgta tgtatgtatc tatctatcat ctatctatct 120
   atcatctatc tatctatcta tctatctatc tatctatcta tctatatccn tttgggatta 180
   tttgtctgga gaatcctgat taacatagtc tgctaacttt tatctgtatc tcctatgtgt 240
   atgcttctcc ttcttcctgt ctctctctct tctttgtcct catttaanct cctttcctgg 300
   gnatattggn aattttgatt ggantctgga cantgtagga gtaaaaattt 350
<210> 60
   <211> 18
   <212> DNA
   <213> Homo sapiens
<400> 60
   tgccaacagc agtcaagc 18
<210> 61
   <211> 21
   <212> DNA
   <213> Homo sapiens
<400> 61
   agcacctgca gcagtaatag c 21
<210> 62
   <211> 196
   <212> DNA
   <213> Homo sapiens
<220>
   <221> misc_feature
   <222> (121)..(121)
   <223> n = a, c, g, or t/u
<220>
   <221> misc_feature
   <222> (170)..(170)
   <223> n = a, c, g, or t/u
<400> 62
   ctgttttatt tgaaaaaaaa aatctgtctc caagaagaaa agttcattct acctgttgcc 60
   aacagcagtc aagcggacat gtttaaaatt ttttaaaaaa gtattttttt ttccaactgg 120
   ngtttaatag cctcattttg gcttttgcta ttactgctgc aggtgctttn atttttttcc 180
   tctgcattat aattac 196
<210> 63
   <211> 25
   <212> DNA
   <213> Homo sapiens
<400> 63
   tccatctatc tttgatgtat ctatg 25
<210> 64
   <211> 25
   <212> DNA
   <213> Homo sapiens
<400> 64
   agttagcaga ctatgttaat cagga 25
<210> 65
   <211> 300
   <212> DNA
   <213> Homo sapiens
<220>
   <221> misc_feature
   <222> (176)..(176)
   <223> n = a, c, g, or t/u
<220>
   <221> misc_feature
   <222> (179)..(179)
   <223> n = a, c, g, or t/u
<220>
   <221> misc_feature
   <222> (189)..(189)
   <223> n = a, c, g, or t/u
<220>
   <221> misc_feature
   <222> (196)..(196)
   <223> n = a, c, g, or t/u
<220>
   <221> misc_feature
   <222> (226).. (226)
   <223> n = a, c, g, or t/u
<220>
   <221> misc_feature
   <222> (238)..(238)
   <223> n = a, c, g, or t/u
<220>
   <221> misc_feature
   <222> (255)..(255)
   <223> n = a, c, g, or t/u
<220>
   <221> misc_feature
   <222> (292)..(292)
   <223> n = a, c, g, or t/u
<400> 65
   aaagctgagt gagccaatac cttgtagtaa atatccatct atctttgatg tatctatgta 60
   tctatctttg tatctatatg tctatgtatc tatgtatgta tgtatctatc tatcatctat 120
   ctatctatca tctatctatc tatctatcta tctatctatc tatctatcta tatccnttng 180
   gtattattng tctggngaat cctgattaac atagtctgct aacttntatc tgtatctnct 240
   atgtgtatgc ttctncttct tcctgtctct ctctctgctt tgtcctcaat tnaaatctcc 300
<210> 66
   <211> 23
   <212> DNA
   <213> Homo sapiens
<400> 66
   ttctgcacaa tttcaataga ttc 23
<210> 67
   <211> 21
   <212> DNA
   <213> Homo sapiens
<400> 67
   gaactgagca aacgagtatg a 21
<210> 68
   <211> 281
   <212> DNA
   <213> Homo sapiens
<400> 68
   agctctgctg gaagagcagg gctgttttct gcacaatttc aatagattcc cctaccctgg 60
   gtttttcagt agatagatag atagatgata gataggtaga tagatagata gatagataga 120
   tagatagata gatagatgat agatagattt tatatatagt atataaaatc tacacacaca 180
   cacacacaca cacacacaca tatttgcctt tccttgacta tcatactcgt ttgctcagtt 240
   cttttttttt ttaaattttt gtttgtaaat ccaaaatgct t 281
<210> 69
   <211> 22
   <212> DNA
   <213> Homo sapiens
<400> 69
   atattcccct atggaagtac ag 22
<210> 70
   <211> 22
   <212> DNA
   <213> Homo sapiens
<400> 70
   aaagttaatt ttcaggcact ct 22
<210> 71
   <211> 381
   <212> DNA
   <213> Homo sapiens
<220>
   <221> misc_feature
   <222> (63)..(63)
   <223> n = a, c, g, or t/u
<220>
   <221> misc_feature
   <222> (270) .. (270)
   <223> n = a, c, g, or t/u
<220>
   <221> misc_feature
   <222> (349)..(349)
   <223> n = a, c, g, or t/u
<220>
   <221> misc_feature
   <222> (371) .. (371)
   <223> n = a, c, g, or t/u
<400> 71
   agctctgtcc ctctagagaa cgctgactaa tatattcccc tatggaagta cagatggttt 60
   ttntaaaata aatttatctg attgtgatga gataatcatc atttttatgt tcagtgtttt 120
   tctaaatttt ttattgttat tgtttttata ctctaaatgg tttttaaata tgcacatatg 180
   tgcatatttt acacacacac acacacacac acacactctc tttatttaga agcattatag 240
   atagagtgcc tgaaaattaa cttttaaccn aagaaaagac aataaggaac aatagggaag 300
   ttatcctttg ctaagggtat ggaaaatatt cacatattat ttataacang ttaaaccaag 360
   tcatgcttga ntataatagc t 381
<210> 72
   <211> 24
   <212> DNA
   <213> Homo sapiens
<400> 72
   gaaattaacc agacactcct aacc 24
<210> 73
   <211> 20
   <212> DNA
   <213> Homo sapiens
<400> 73
   cttagaattg cctttgctgc 20
<210> 74
   <211> 465
   <212> DNA
   <213> Homo sapiens
<220>
   <221> misc_feature
   <222> (70)..(70)
   <223> n = a, c, g, or t/u
<220>
   <221> misc_feature
   <222> (142)..(142)
   <223> n = a, c, g, or t/u
<220>
   <221> misc_feature
   <222> (282) .. (282)
   <223> n = a, c, g, or t/u
<220>
   <221> misc_feature
   <222> (289)..(289)
   <223> n = a, c, g, or t/u
<220>
   <221> misc_feature
   <222> (301) .. (301)
   <223> n = a, c, g, or t/u
<220>
   <221> misc_feature
   <222> (306)..(306)
   <223> n = a, c, g, or t/u
<220>
   <221> misc_feature
   <222> (319)..(320)
   <223> n = a, c, g, or t/u
<220>
   <221> misc_feature
   <222> (335)..(335)
   <223> n = a, c, g, or t/u
<220>
   <221> misc_feature
   <222> (338)..(338)
   <223> n = a, c, g, or t/u
<220>
   <221> misc_feature
   <222> (344)..(344)
   <223> n = a, c, g, or t/u
<220>
   <221> misc_feature
   <222> (347)..(348)
   <223> n = a, c, g, or t/u
<220>
   <221> misc_feature
   <222> (350) .. (351)
   <223> n = a, c, g, or t/u
<220>
   <221> misc_feature
   <222> (353)..(353)
   <223> n = a, c, g, or t/u
<220>
   <221> misc_feature
   <222> (357)..(358)
   <223> n = a, c, g, or t/u
<220>
   <221> misc_feature
   <222> (360)..(360)
   <223> n = a, c, g, or t/u
<220>
   <221> misc_feature
   <222> (363)..(363)
   <223> n = a, c, g, or t/u
<220>
   <221> misc_feature
   <222> (365)..(365)
   <223> n = a, c, g, or t/u
<220>
   <221> misc_feature
   <222> (370)..(370)
   <223> n = a, c, g, or t/u
<220>
   <221> misc_feature
   <222> (372)..(372)
   <223> n = a, c, g, or t/u
<220>
   <221> misc_feature
   <222> (378)..(379)
   <223> n = a, c, g, or t/u
<220>
   <221> misc_feature
   <222> (381) .. (381)
   <223> n = a, c, g, or t/u
<220>
   <221> misc_feature
   <222> (387)..(388)
   <223> n = a, c, g, or t/u
<220>
   <221> misc_feature
   <222> (391) .. (395)
   <223> n = a, c, g, or t/u
<220>
   <221> misc_feature
   <222> (397)..(401)
   <223> n = a, c, g, or t/u
<220>
   <221> misc_feature
   <222> (403)..(403)
   <223> n = a, c, g, or t/u
<220>
   <221> misc_feature
   <222> (405) .. (409)
   <223> n = a, c, g, or t/u
<220>
   <221> misc_feature
   <222> (412)..(412)
   <223> n = a, c, g, or t/u
<220>
   <221> misc_feature
   <222> (416)..(423)
   <223> n = a, c, g, or t/u
<220>
   <221> misc_feature
   <222> (425)..(425)
   <223> n = a, c, g, or t/u
<220>
   <221> misc_feature
   <222> (427)..(430)
   <223> n = a, c, g, or t/u
<220>
   <221> misc_feature
   <222> (432)..(440)
   <223> n = a, c, g, or t/u
<220>
   <221> misc_feature
   <222> (442)..(454)
   <223> n = a, c, g, or t/u
<220>
   <221> misc_feature
   <222> (457)..(461)
   <223> n = a, c, g, or t/u
<220>
   <221> misc_feature
   <222> (465).. (465)
   <223> n = a, c, g, or t/u
<400> 74
   gaataaaaat atgaggtatt agaaatttac agataggaag aaattaacca gacactccta 60
   accaccgatn agtttaaaga ggagatagat agatagatga tagatagata gatagataga 120
   tagataccac tgaaaatgca ancacaaatt agcagattat atgtgatgca gcaaaggcaa 180
   ttctaagtag attctaactg ctacattgat agcagtaccc actgacatta ccggaaagga 240
   tggtatccat aaccacctac ctatatacct ccgcagctgg anattaggnt taagcttctt 300
   ngggcncctg gcggccccnn ttgtggtccc cggtnggncc ccgnttnngn ntngctnngn 360
   ttncnttggn gncccccnnt nggtttnngg nnnnntnnnn ntngnnnnnt tncccnnnnn 420
   nnntntnnnn cnnnnnnnnn tnnnnnnnnn nnnnggnnnn ngggn 465
<210> 75
   <211> 23
   <212> DNA
   <213> Homo sapiens
<400> 75
   gcaacaaccc taatgagtat acg 23
<210> 76
   <211> 20
   <212> DNA
   <213> Homo sapiens
<400> 76
   gagtctcacc agggcttaca 20
<210> 77
   <211> 355
   <212> DNA
   <213> Homo sapiens
<220>
   <221> misc_feature
   <222> (254)..(254)
   <223> n = a, c, g, or t/u
<220>
   <221> misc_feature
   <222> (342)..(342)
   <223> n = a, c, g, or t/u
<220>
   <221> misc_feature
   <222> (350)..(350)
   <223> n = a, c, g, or t/u
<220>
   <221> misc_feature
   <222> (354)..(354)
   <223> n = a, c, g, or t/u
<400> 77
   aaagctgtct ccttttgtaa agtgtgctca gaggaatctt tttcagtaaa taaagtctgc      60
   acccagacat ctcactttgt ataccacgga gaatttacca tgactcttct cagtgataaa     120
   cgtcaatata gaataatcag gagaaaaaga gaaatccagt aaagaaataa gtctgtagaa     180
   agcaacaacc ctaatgagta tacgatatct atctatctat ctatctatct atctatctat     240
   ctatctatct atgnatctat ctatctaaca ttatataaaa tatatatttc ctcctgtatt     300
   ggggccctgt gtgtaagccc tggtgagact caaaaatttg antattcctn tttnt          355
<210> 78
   <211> 20
   <212> DNA
   <213> Homo sapiens
<400> 78
   tcctctcatc tcggatatgg 20
<210> 79
   <211> 22
   <212> DNA
   <213> Homo sapiens
<400> 79
   aaggctttca gacttacact gg 22
<210> 80
   <211> 614
   <212> DNA
   <213> Homo sapiens
<220>
   <221> misc_feature
   <222> (49) .. (49)
   <223> n = a, c, g, or t/u
<220>
   <221> misc_feature
   <222> (51) .. (51)
   <223> n = a, c, g, or t/u
<220>
   <221> misc_feature
   <222> (133)..(133)
   <223> n = a, c, g, or t/u
<220>
   <221> mise_feature
   <222> (148)..(148)
   <223> n = a, c, g, or t/u
<220>
   <221> misc_feature
   <222> (192)..(192)
   <223> n = a, c, g, or t/u
<220>
   <221> misc_feature
   <222> (250)..(250)
   <223> n = a, c, g, or t/u
<220>
   <221> misc_feature
   <222> (256) .. (256)
   <223> n = a, c, g, or t/u
<220>
   <221> misc_feature
   <222> (265) .. (265)
   <223> n = a, c, g, or t/u
<220>
   <221> misc_feature
   <222> (268)..(268)
   <223> n = a, c, g, or t/u
<220>
   <221> misc_feature
   <222> (270)..(270)
   <223> n = a, c, g, or t/u
<220>
   <221> misc_feature
   <222> (297)..(297)
   <223> n = a, c, g, or t/u
<220>
   <221> misc_feature
   <222> (307)..(307)
   <223> n = a, c, g, or t/u
<220>
   <221> misc_feature
   <222> (310)..(310)
   <223> n = a, c, g, or t/u
<220>
   <221> misc_feature
   <222> (314)..(314)
   <223> n = a, c, g, or t/u
<220>
   <221> misc_feature
   <222> (317)..(317)
   <223> n = a, c, g, or t/u
<220>
   <221> misc_feature
   <222> (321)..(321)
   <223> n = a, c, g, or t/u
<220>
   <221> misc_feature
   <222> (323)..(323)
   <223> n = a, c, g, or t/u
<220>
   <221> misc_feature
   <222> (326)..(326)
   <223> n = a, c, g, or t/u
<220>
   <221> misc_feature
   <222> (331)..(331)
   <223> n = a, c, g, or t/u
<220>
   <221> misc_feature
   <222> (338)..(338)
   <223> n = a, c, g, or t/u
<220>
   <221> misc_feature
   <222> (346)..(346)
   <223> n = a, c, g, or t/u
<220>
   <221> misc_feature
   <222> (355)..(355)
   <223> n = a, c, g, or t/u
<220>
   <221> misc_feature
   <222> (359)..(359)
   <223> n = a, c, g, or t/u
<220>
   <221> misc_feature
   <222> (365)..(365)
   <223> n = a, c, g, or t/u
<220>
   <221> misc_feature
   <222> (377)..(377)
   <223> n = a, c, g, or t/u
<220>
   <221> misc_feature
   <222> (383)..(383)
   <223> n = a, c, g, or t/u
<220>
   <221> misc_feature
   <222> (389)..(389)
   <223> n = a, c, g, or t/u
<220>
   <221> misc_feature
   <222> (396)..(396)
   <223> n = a, c, g, or t/u
<220>
   <221> misc_feature
   <222> (402)..(402)
   <223> n = a, c, g, or t/u
<220>
   <221> misc_feature
   <222> (410)..(410)
   <223> n = a, c, g, or t/u
<220>
   <221> misc_feature
   <222> (419).. (419)
   <223> n = a, c, g, or t/u
<220>
   <221> misc_feature
   <222> (427)..(427)
   <223> n = a, c, g, or t/u
<220>
   <221> misc_feature
   <222> (488)..(488)
   <223> n = a, c, g, or t/u
<220>
   <221> misc_feature
   <222> (493)..(493)
   <223> n = a, c, g, or t/u
<220>
   <221> misc_feature
   <222> (591)..(591)
   <223> n = a, c, g, or t/u
<220>
   <221> misc_feature
   <222> (593)..(593)
   <223> n=a, c, g, or t/u
<220>
   <221> misc_feature
   <222> (595)..(595)
   <223> n = a, c, g, or t/u
<220>
   <221> misc_feature
   <222> (603)..(603)
   <223> n = a, c, g, or t/u
<400> 80
   ttatttattc actcattcaa taaatattta tgaatttcct ttaatggcna ngaaagtatg 60
   tttggtactg aatatggtga gcaagatttt cctctcatct cggatatgga aagatcttgg 120
   aaatcattat acntcatact tacaatanga aagaagctga gcaatttgaa aatcaacaat 180
   ttcttttgta cntgtcagaa aagtgaagat atattaatca gggttcttca gagaaacata 240
   accaataggn cacagntcta tatgnccncn tttatctatc tatctatcta tctatcncta 300
   tctatcncan accnggngaa ntnatntttg ngagattnat gcaagnctga gaaanaccna 360
   agaanctgct ccctgtnaaa ctngagatnc aagaanctga anagtatagn tccagtccna 420
   agtctanaga ccttagaatt aggaaaactg atactataaa taccagtgta agtctgaaag 480
   ccttaaanac canatagtgc cattgaaagg gcagaagact gatgtcccag ttcaagcagg 540
   caaagttaga gaagccttat tttctgcaac attgttctat tcagaccctt nanangattg 600
   acnatgtcca ccca 614
<210> 81
   <211> 23
   <212> DNA
   <213> Homo sapiens
<400> 81
   tctccaagct attgattgga taa 23
<210> 82
   <211> 27
   <212> DNA
   <213> Homo sapiens
<400> 82
   ttaagagcca atttatataa aagcagc 27
<210> 83
   <211> 252
   <212> DNA
   <213> Homo sapiens
<400> 83
   ccccttttgc caacgccatg cttcacgtag ggagcctgac atgcagaaaa ctctccaagc 60
   tattgattgg ataaagagcc agagctgact gaattccatt cttcttgagc ctctcattct 120
   gtgtttctcg aatttttacc aaagcatctt gacacacaaa tatctgactc aaggaaaagg 180
   aaaaacaact gctttttctc cagctgcttt tatataaatt ggctcttaaa ctttctaagt 240
   ttattatgga ta 252
<210> 84
   <211> 23
   <212> DNA
   <213> Homo sapiens
<400> 84
   atgcttcagg atgacgtaat aca 23
<210> 85
   <211> 20
   <212> DNA
   <213> Homo sapiens
<400> 85
   aaattctcgc tgattggagg 20
<210> 86
   <211> 166
   <212> DNA
   <213> Homo sapiens
<220>
   <221> misc_feature
   <222> (49)..(49)
   <223> n = a, c, g, or t/u
<400> 86
   ctagtaccat aatccctttt ggaataaacc atcccacctt tagtcaganc agatgcttca 60
   ggatgacgta atacataata agcctactca gttctactct ggctttgtat gtcttcaaag 120
   tgatattttt ttaagtatta cttgtccctc caatcagcga gaattt 166
<210> 87
   <211> 24
   <212> DNA
   <213> Homo sapiens
<400> 87
   tcaaacttat aactgcagag aacg 24
<210> 88
   <211> 20
   <212> DNA
   <213> Homo sapiens
<400> 88
   atggtaaacc ctccccatta 20
<210> 89
   <211> 476
   <212> DNA
   <213> Homo sapiens
<220>
   <221> misc_feature
   <222> (221)..(221)
   <223> n = a, c, g, or t/u
<220>
   <221> misc_feature
   <222> (228)..(228)
   <223> n = a, c, g, or t/u
<220>
   <221> misc_feature
   <222> (255)..(255)
   <223> n = a, c, g, or t/u
<220>
   <221> misc_feature
   <222> (258)..(258)
   <223> n = a, c, g, or t/u
<220>
   <221> misc_feature
   <222> (275)..(275)
   <223> n = a, c, g, or t/u
<220>
   <221> misc_feature
   <222> (279)..(279)
   <223> n = a, c, g, or t/u
<220>
   <221> misc_feature
   <222> (287)..(287)
   <223> n = a, c, g, or t/u
<220>
   <221> misc_feature
   <222> (292)..(292)
   <223> n = a, c, g, or t/u
<220>
   <221> misc_feature
   <222> (299).. (299)
   <223> n = a, c, g, or t/u
<220>
   <221> misc_feature
   <222> (308)..(308)
   <223> n = a, c, g, or t/u
<220>
   <221> misc_feature
   <222> (318) .. (318)
   <223> n = a, c, g, or t/u
<220>
   <221> misc_feature
   <222> (325) .. (325)
   <223> n = a, c, g, or t/u
<220>
   <221> misc_feature
   <222> (327)..(327)
   <223> n = a, c, g, or t/u
<220>
   <221> misc_feature
   <222> (331)..(334)
   <223> n = a, c, g, or t/u
<220>
   <221> misc_feature
   <222> (338) .. (339)
   <223> n = a, c, g, or t/u
<220>
   <221> misc_feature
   <222> (341)..(343)
   <223> n = a, c, g, or t/u
<220>
   <221> misc_feature
   <222> (351)..(351)
   <223> n = a, c, g, or t/u
<220>
   <221> misc_feature
   <222> (358)..(358)
   <223> n = a, c, g, or t/u
<220>
   <221> misc_feature
   <222> (362)..(362)
   <223> n = a, c, g, or t/u
<220>
   <221> misc_feature
   <222> (375)..(375)
   <223> n = a, c, g, or t/u
<220>
   <221> misc_feature
   <222> (377)..(377)
   <223> n = a, c, g, or t/u
<220>
   <221> misc_feature
   <222> (385)..(385)
   <223> n = a, c, g, or t/u
<220>
   <221> misc_feature
   <222> (395)..(396)
   <223> n = a, c, g, or t/u
<220>
   <221> misc_feature
   <222> (399)..(399)
   <223> n = a, c, g, or t/u
<220>
   <221> misc_feature
   <222> (402)..(402)
   <223> n = a, c, g, or t/u
<220>
   <221> misc_feature
   <222> (406)..(406)
   <223> n = a, c, g, or t/u
<220>
   <221> misc_feature
   <222> (409)..(411)
   <223> n = a, c, g, or t/u
<220>
   <221> misc_feature
   <222> (414)..(414)
   <223> n = a, c, g, or t/u
<220>
   <221> misc_feature
   <222> (416)..(417)
   <223> n = a, c, g, or t/u
<220>
   <221> misc_feature
   <222> (419)..(419)
   <223> n = a, c, g, or t/u
<220>
   <221> misc_feature
   <222> (426)..(426)
   <223> n = a, c, g, or t/u
<220>
   <221> misc_feature
   <222> (435)..(435)
   <223> n = a, c, g, or t/u
<220>
   <221> misc_feature
   <222> (437)..(437)
   <223> n = a, c, g, or t/u
<220>
   <221> misc_feature
   <222> (439) .. (439)
   <223> n = a, c, g, or t/u
<220>
   <221> misc_feature
   <222> (442)..(442)
   <223> n = a, c, g, or t/u
<220>
   <221> misc_feature
   <222> (446)..(446)
   <223> n = a, c, g, or t/u
<220>
   <221> misc_feature
   <222> (448)..(448)
   <223> n = a, c, g, or t/u
<220>
   <221> misc_feature
   <222> (453)..(453)
   <223> n = a, c, g, or t/u
<220>
   <221> misc_feature
   <222> (458)..(458)
   <223> n = a, c, g, or t/u
<220>
   <221> misc_feature
   <222> (462)..(462)
   <223> n = a, c, g, or t/u
<220>
   <221> misc_feature
   <222> (468)..(468)
   <223> n = a, c, g, or t/u
<220>
   <221> misc_feature
   <222> (472)..(473)
   <223> n = a, c, g, or t/u
<400> 89
   aagactgcaa ttacatttgc atcaaactta taactgcaga gaacgttgcc cactatttta 60
   taccacacaa cagtattctt agccagatta catctatcta tctatctatc tatctatcta 120
   tctatctatc tatctatcta tcatctatct agctagctat ctatctatag aactaatggg 180
   gagggtttac catgtttggg tgaacccaaa cattttatgg ncaagggntt ggaaaattac 240
   ccttatctac aactnttnaa cttgttttgg taggngtgnt aattccntgg gnttggaana 300
   acttttgnaa tttcctcntt gtttntnatt nnnnattnnt nnncattatt ntggggtntt 360
   cngggtggag ggctnanttt ggccncccgg gtccnnggng cnagtnggnn nggntnntng 420
   ggtttncttg ggaancntnc cncctncngg ggnttcangg gntttttntt tnnttg 476
<210> 90
   <211> 23
   <212> DNA
   <213> Homo sapiens
<400> 90
   ctttgcaatc ttagttaatt ggc 23
<210> 91
   <211> 413
   <212> DNA
   <213> Homo sapiens
<220>
   <221> misc_feature
   <222> (153)..(153)
   <223> n = a, c, g, or t/u
<220>
   <221> misc_feature
   <222> (182)..(182)
   <223> n = a, c, g, or t/u
<220>
   <221> misc_feature
   <222> (184)..(184)
   <223> n = a, c, g, or t/u
<220>
   <221> misc_feature
   <222> (205)..(205)
   <223> n = a, c, g, or t/u
<220>
   <221> misc_feature
   <222> (207)..(207)
   <223> n = a, c, g, or t/u
<220>
   <221> misc_feature
   <222> (213)..(213)
   <223> n = a, c, g, or t/u
<220>
   <221> misc_feature
   <222> (224)..(224)
   <223> n = a, c, g, or t/u
<220>
   <221> misc_feature
   <222> (227)..(227)
   <223> n = a, c, g, or t/u
<220>
   <221> misc_feature
   <222> (241).. (241)
   <223> n = a, c, g, or t/u
<220>
   <221> misc_feature
   <222> (243)..(243)
   <223>n = a, c, g, or t/u
<220>
   <221> misc_feature
   <222> (258)..(258)
   <223> n = a, c, g, or t/u
<220>
   <221> misc_feature
   <222> (282)..(282)
   <223> n = a, c, g, or t/u
<220>
   <221> misc_feature
   <222> (294)..(294)
   <223> n = a, c, g, or t/u
<220>
   <221> misc_feature
   <222> (298)..(298)
   <223> n = a, c, g, or t/u
<220>
   <221> misc_feature
   <222> (302)..(302)
   <223> n = a, c, g, or t/u
<220>
   <221> misc_feature
   <222> (304)..(304)
   <223> n = a, c, g, or t/u
<220>
   <221> misc_feature
   <222> (307)..(308)
   <223> n = a, c, g, or t/u
<220>
   <221> misc_feature
   <222> (310)..(311)
   <223> n = a, c, g, or t/u
<220>
   <221> misc_feature
   <222> (313)..(313)
   <223> n = a, c, g, or t/u
<220>
   <221> misc_feature
   <222> (315)..(316)
   <223> n = a, c, g, or t/u
<220>
   <221> misc_feature
   <222> (318)..(319)
   <223> n = a, c, g, or t/u
<220>
   <221> mise_feature
   <222> (322)..(324)
   <223> n = a, c, g, or t/u
<220>
   <221> misc_feature
   <222> (326)..(326)
   <223> n = a, c, g, or t/u
<220>
   <221> misc_feature
   <222> (328)..(328)
   <223> n = a, c, g, or t/u
<220>
   <221> misc_feature
   <222> (335)..(335)
   <223> n = a, c, g, or t/u
<220>
   <221> misc_feature
   <222> (340)..(340)
   <223> n = a, c, g, or t/u
<220>
   <221> misc_feature
   <222> (354)..(356)
   <223> n = a, c, g, or t/u
<220>
   <221> misc_feature
   <222> (371)..(371)
   <223> n = a, c, g, or t/u
<220>
   <221> misc_feature
   <222> (388)..(388)
   <223> n = a, c, g, or t/u
<220>
   <221> misc_feature
   <222> (394)..(396)
   <223> n = a, c, g, or t/u
<220>
   <221> misc_feature
   <222> (398)..(398)
   <223> n = a, c, g, or t/u
<220>
   <221> misc_feature
   <222> (405)..(405)
   <223> n = a, c, g, or t/u
<400> 91
   agatgtttaa ctttgcaatc ttagttaatt ggcagaaatg aaatttagtt tccacaactt      60
   ttattcgata ttaaaacacc accaccatca gcagcagcag cagcagcagc agcatcgctg     120
   ttactccata tcatagttca gagcatttaa agnggtcaaa atatacaact aggctgacac     180
   cngnataagg tttaatttta aaccngnggt ctnccctcta aggnggnttt ttttttcttg    240
   ncntggcttc tttttccntt tgcttttgta aaatatcaag gnatttttgg gttnttcntg     300
   gnanttnncn nantnntnnt tnnncncncc ccccntttgn ggcgggggtc cccnnnttgc     360
   cccggggttg ngtgcagtag gggggtcncg ggtnnngnaa gtttnggggc cct            413
<210> 92
   <211> 20
   <212> DNA
   <213> Homo sapiens
<400> 92
   ttctgccttt cctgactgtc 20
<210> 93
   <211> 20
   <212> DNA
   <213> Homo sapiens
<400> 93
   tgtttcccat gtcttgatga 20
<210> 94
   <211> 244
   <212> DNA
   <213> Homo sapiens
<400> 94
   ctactaagca aattctgctc agccttctgc ctttcctgac tgtcttgttg gcccttccca 60
   ctttaaggat gcctgtttaa gtagccacct ctaattagga atcttccctt gttctttctc 120
   aggaggctta gacactgtca gtttcctgaa gacagaaaat aagcctgcat tatcctagta 180
   gtggattcaa aactaattgt gtcctgagtc tttcaatcat caagacatgg gaaacactca 240
   acag 244
<210> 95
   <211> 25
   <212> DNA
   <213> Homo sapiens
<400> 95
   ccagtaatta gacattgaca ggttc 25
<210> 96
   <211> 26
   <212> DNA
   <213> Homo sapiens
<400> 96
   ttttactaga caggcttgat aaacaa 26
<210> 97
   <211> 328
   <212> DNA
   <213> Homo sapiens
<400> 97
   ccagtaatta gacattgaca ggttccatac tagtaatgta gggaataggg ctgctgcttt 60
   ttgggtttcc ttgagtatac tttgtgctgc ataaatatgg caatggatag taaataattt 120
   gtatgcagac ctttagtgtc gattaacctg tgaataaggg aacaacaatc aaggacaaaa 180
   atcaaaagac taattctcta tacattttga gcttttgtaa aaaagtaaga ttagctgaat 240
   atatctgaaa aatttctaat ctcctttaca attttttaaa ttgtttatca agcctgtcta 300
   gtaaaaataa ttcagtttcg gaatgtgg 328
<210> 98
   <211> 23
   <212> DNA
   <213> Homo sapiens
<400> 98
   ggacatcctt gatttgctca taa 23
<210> 99
   <211> 22
   <212> DNA
   <213> Homo sapiens
<400> 99
   gattgactga aaacaggcac at 22
<210> 100
   <211> 337
   <212> DNA
   <213> Homo sapiens
<220>
   <221> misc_feature
   <222> (96)..(96)
   <223> n = a, c, g, or t/u
<220>
   <221> misc_feature
   <222> (140)..(140)
   <223> n = a, c, g, or t/u
<220>
   <221> misc_feature
   <222> (309) .. (309)
   <223> n = a, c, g, or t/u
<400> 100
   ggacatcctt gatttgctca taatacactc attcctttca ccattgagtg tgcacatatt     60
   tctctgattg gaaagaacta cagaggaggt tttacntttt actttccagt ttgctattaa    120
   agagagaaaa ctaacagagn gaaatcaagc aactcaaaac aaccttacac acacacacac    180
   acacacacac tcacaaagat attttgttca ccatatgtat tgatgtgcct gttttcagtc    240
   aatccacagg aagggctaag gagagtgaca tctgggctac attaaaagga cagtcacatt    300
   gctcaaagna ctcaagttta gcccgagtac agtagct                              337
<210> 101
   <211> 20
   <212> DNA
   <213> Homo sapiens
<400> 101
   agcttgcaga tagcctgcta 20
<210> 102
   <211> 25
   <212> DNA
   <213> Homo sapiens
<400> 102
   tacggtaggt aggtagatag attcg 25
<210> 103
   <211> 293
   <212> DNA
   <213> Homo sapiens
<220>
   <221> misc_feature
   <222> (14)..(14)
   <223> n = a, c, g, or t/u
<220>
   <221> misc_feature
   <222> (293)..(293)
   <223> n = a, c, g, or t/u
<400> 103
   ctctacagtc tctnaccttt ggactccagg actttcacca gcaccctcaa cattcccact 60
   gggttctcag gactttatag ttgtactgag ccatgccact ggatcctagg gtctccagct 120
   tgcagatagc ctgctatggg acttaatctt tgtaataagg tgagtcaatt ctgccaataa 180
   acctactttc atctctatct atctatctat ctatctatct atctatctat atctatcatc 240
   tatctatcga atctatctac ctacctaccg tattagttct gtctctctgg agn 293
<210> 104
   <211> 23
   <212> DNA
   <213> Homo sapiens
<400> 104
   tgagagaacg aaatctctat cgg 23
<210> 105
   <211> 23
   <212> DNA
   <213> Homo sapiens
<400> 105
   aggcagcaag tttttataaa ggc 23
<210> 106
   <211> 331
   <212> DNA
   <213> Homo sapiens
<400> 106
   atgtatctat cccaattgag tcagctagaa acagttgact gactaaatgg aaactagtct 60
   atttgacaaa gtctttctgt gttggtgtct actgaagtta tagtttaccc ttcctaaaaa 120
   tgaaaagttt gtttcatata gtgagagaac gaaatctcta tcggccagtc agatgtttct 180
   catccttctt gctctgcctt tgagttgttc cgtgatcatt ctgaataagc atttgccttt 240
   ataaaaactt gctgcctgac taaagattaa caggttatag tttaaatttg taattaattc 300
   taccatcttg caataaagtg acaattgaat g 331
<210> 107
   <211> 22
   <212> DNA
   <213> Homo sapiens
<400> 107
   acactgtagc agaggcttga cc 22
<210> 108
   <211> 20
   <212> DNA
   <213> Homo sapiens
<400> 108
   aggccaagtt atgtgccacc 20
<210> 109
   <211> 371
   <212> DNA
   <213> Homo sapiens
<220>
   <221> misc_feature
   <222> (345)..(345)
   <223> n = a, c, g, or t/u
<400> 109
   aaatgacact ttaaggaggt aacactgtag cagaggcttg accaccaccc agttctcact 60
   agcactgagg atgctctatt ggttgggtta cccacacacg catagacatg cacacacaca 120
   gacacacaga cacacacaca cacacacaca caccagatat agcattccaa accatcaata 180
   tgctatgcaa tactgcatta acaggtcatg cctgtggtgg cacataactt ggcctagaaa 240
   atactgggga cgtctgcatt cccttttatt atcgaattga cttacttggc ttctgagttt 300
   tcctcagaag taatacttca atacctcttc catttctgcc ttgancattg tttggggtac 360
   caagtatagc t 371
<210> 110
   <211> 25
   <212> DNA
   <213> Homo sapiens
<400> 110
   ctctcaaagt aagagcgatg ttgta 25
<210> 111
   <211> 21
   <212> DNA
   <213> Homo sapiens
<400> 111
   ccgaagtaga aaatcttggc a 21
<210> 112
   <211> 358
   <212> DNA
   <213> Homo sapiens
<220>
   <221> misc_feature
   <222> (50)..(50)
   <223> n = a, c, g, or t/u
<220>
   <221> misc_feature
   <222> (56)..(56)
   <223> n = a, c, g, or t/u
<220>
   <221> misc_feature
   <222> (223)..(223)
   <223> n = a, c, g, or t/u
<220>
   <221> misc_feature
   <222> (249)..(249)
   <223> n = a, c, g, or t/u
<220>
   <221> misc_feature
   <222> (273)..(273)
   <223> n = a, c, g, or t/u
<220>
   <221> misc_feature
   <222> (315)..(315)
   <223> n = a, c, g, or t/u
<220>
   <221> misc_feature
   <222> (323)..(323)
   <223> n = a, c, g, or t/u
<220>
   <221> misc_feature
   <222> (328)..(328)
   <223> n = a, c, g, or t/u
<220>
   <221> misc_feature
   <222> (345)..(345)
   <223> n = a, c, g, or t/u
<400> 112
   agctctcaaa gtaagagcga tgttgtaact gactgagttg ttttgtgaan ttttgntttt 60
   ggagtcagtg gagcatgtta ttagatgtaa atttaaacac acacacacac acacacacac 120
   acacacacga gaagtaagtg ccaagatttt ctacttcggc gcctatattt ctatatactg 180
   attttctgta tttcccagac ttgaatatag attgtctttc tgntttatca tagacaatct 240
   cataataant taggcataat aaggtaatga ggnttttctg ggcttctttt catcatccct 300
   gcaatttgag tctcntttat agntgaantc ttctctgtaa taacntcttg ttttagct 358
<210> 113
   <211> 23
   <212> DNA
   <213> Homo sapiens
<400> 113
   atttctaaga ggactcccaa act 23
<210> 114
   <211> 22
   <212> DNA
   <213> Homo sapiens
<400> 114
   atattttgaa actcaggagc at 22
<210> 115
   <211> 278
   <212> DNA
   <213> Homo sapiens
<220>
   <221> misc_feature
   <222> (33) .. (33)
   <223> n = a, c, g, or t/u
<220>
   <221> misc_feature
   <222> (75)..(75)
   <223> n = a, c, g, or t/u
<220>
   <221> misc_feature
   <222> (100)..(100)
   <223> n = a, c, g, or t/u
<220>
   <221> misc_feature
   <222> (102)..(102)
   <223> n = a, c, g, or t/u
<220>
   <221> misc_feature
   <222> (108)..(108)
   <223> n = a, c, g, or t/u
<220>
   <221> misc_feature
   <222> (163)..(163)
   <223> n = a, c, g, or t/u
<220>
   <221> misc_feature
   <222> (177)..(177)
   <223> n = a, c, g, or t/u
<220>
   <221> misc_feature
   <222> (265)..(265)
   <223> n = a, c, g, or t/u
<400> 115
   cgtcttacca aaccaacata atatagcaat ggnaaccaaa aatttctaag aggactccca 60
   aactacattc ttctncctga attaaataca ggcattcaan anaaacanac acacacacac 120
   acacacacac acacacacac acacacgcac acccttcaaa tcntagcata aattccnctt 180
   atataaacat aaccatgctc ctgagtttca aaatattggg tggttcgaag ttcgaagcaa 240
   caaatttcca gttagtgtct attanttgtt ggacagct 278
<210> 116
   <211> 20
   <212> DNA
   <213> Homo sapiens
<400> 116
   gagccatgtt catgccactg 20
<210> 117
   <211> 20
   <212> DNA
   <213> Homo sapiens
<400> 117
   caaacccgac taccagcaac 20
<210> 118
   <211> 684
   <212> DNA
   <213> Homo sapiens
<220>
   <221> misc_feature
   <222> (9)..(9)
   <223> n = a, c, g, or t/u
<220>
   <221> misc_feature
   <222> (75) .. (75)
   <223> n = a, c, g, or t/u
<220>
   <221> misc_feature
   <222> (263)..(263)
   <223> n = a, c, g, or t/u
<220>
   <221> misc_feature
   <222> (306)..(306)
   <223> n = a, c, g, or t/u
<220>
   <221> misc_feature
   <222> (309) .. (309)
   <223> n = a, c, g, or t/u
<220>
   <221> misc_feature
   <222> (313) .. (313)
   <223> n = a, c, g, or t/u
<220>
   <221> misc_feature
   <222> (534)..(534)
   <223> n = a, c, g, or t/u
<400> 118
   aattgagcnc aggagtttaa gaccagcctg ggtaacacag tgagacccct gtctctacaa     60
   aaaaatacaa aaatnagttg ggcatggtgg cacgtgcctg tagtctcagc tacttgcagg    120
   gctgaggcag gaggagttct tgagcccaga aggttaaggc tgcagtgagc catgttcatg    180
   ccactgcact tcactctgag tgacaaattg agaccttgtc tcagaaagaa agaaagaaag    240
   aaagaaagaa agaaagaaag aangaaagaa agaaagtaag aaaaagagag ggaaagaaag    300
   agaaanagna aanaaatagt agcaactgtt attgtaagac atctccacac accagagaag    360
   ttaattttaa ttttaacatg ttaagaacag agagaagcca acatgtccac cttaggctga    420
   cggtttgttt atttgtgttg ttgctggtag tcgggtttgt tatttttaaa gtagcttatc    480
   caatacttca ttaacaattt cagtaagtta tttcatcttt caacataaat acgnacaagg    540
   atttcttctg gtcaagacca aactaatatt agtccatagt aggagctaat actatcacat    600
   ttactaagta ttctatttgc aatttgactg tagcccatag ccttttgtcg gctaaagtga    660
   gcttaatgct gatcgactct agag                                           684
<210> 119
   <211> 19
   <212> DNA
   <213> Homo sapiens
<400> 119
   tggaggttgc aatgagctg 19
<210> 120
   <211> 19
   <212> DNA
   <213> Homo sapiens
<400> 120
   catgcacacc taattggcg 19
<210> 121
   <211> 158
   <212> DNA
   <213> Homo sapiens
<220>
   <221> misc_feature
   <222> (51)..(51)
   <223> n = a, c, g, or t/u
<220>
   <221> misc_feature
   <222> (60)..(60)
   <223> n = a, c, g, or t/u
<220>
   <221> misc_feature
   <222> (68)..(68)
   <223> n = a, c, g, or t/u
<220>
   <221> misc_feature
   <222> (91)..(91)
   <223> n = a, c, g, or t/u
<220>
   <221> misc_feature
   <222> (150)..(150)
   <223> n = a, c, g, or t/u
<220>
   <221> misc_feature
   <222> (155)..(155)
   <223> n = a, c, g, or t/u
<400> 121
   acgaggacag gagttcaaga ccagcctggc caacatggtg aaccccgttt ntactaaaan 60
   tacaaaantt ggtcgggagg ctggggcagg ngacatgctt gaccccagga ggtggaggtt 120
   gcaatgagct gagattgcac cactgcactn cagcntgg 158
<210> 122
   <211> 136
   <212> DNA
   <213> Homo sapiens
<220>
   <221> misc_feature
   <222> (16)..(16)
   <223> n = a, c, g, or t/u
<220>
   <221> mise_feature
   <222> (19)..(20)
   <223> n = a, c, g, or t/u
<220>
   <221> misc_feature
   <222> (24) .. (24)
   <223> n = a, c, g, or t/u
<220>
   <221> mise_feature
   <222> (27)..(28)
   <223> n = a, c, g, or t/u
<220>
   <221> misc_feature
   <222> (30).. (31)
   <223> n = a, c, g, or t/u
<220>
   <221> misc_feature
   <222> (41) .. (41)
   <223> n = a, c, g, or t/u
<220>
   <221> misc_feature
   <222> (92)..(92)
   <223> n = a, c, g, or t/u
<400> 122
   aagaaagaga aagganagnn aggnagnnan naaactacat ntgaagtcaa cactagtatt 60
   ggtgggagag gaattttatg ctgcattccc cnacaaccac tagatacgcc aattaggtgt 120
   gcatggtcca tgctat 136
<210> 123
   <211> 20
   <212> DNA
   <213> Homo sapiens
<400> 123
   gcaaagcaga agtgagcatg 20
<210> 124
   <211> 19
   <212> DNA
   <213> Homo sapiens
<400> 124
   taggactaca ggcgtgtgc 19
<210> 125
   <211> 410
   <212> DNA
   <213> Homo sapiens
<220>
   <221> misc_feature
   <222> (62) .. (62)
   <223> n = a, c, g, or t/u
<220>
   <221> misc_feature
   <222> (386)..(386)
   <223> n = a, c, g, or t/u
<220>
   <221> misc_feature
   <222> (397) .. (398)
   <223> n = a, c, g, or t/u
<400> 125
   catatgggtc cacaagcaac ctcagtcctt gtctcttcag aagaaagaat tctactgagg 60
   gncataaggc agaaggagag acctaggcaa gttgcaaagc agaagtgagc atgtattaaa 120
   aagctttaga acagtaagga aaggaagaaa agaaaagaag gaaagttcaa cttggaagag 180
   ggccaagccg gcaacttggc agaaggattg cttgagccca ggagttaaga ccagtctggg 240
   caatatagtg agactccatc tctgcataca tacatacata catacataca tacatacata 300
   catacatatt gcagggtatg atggcacacg cctgtagtcc tagctactct ggaggttgag 360
   atgggagggt cactgagcct gggaanttga ggctgcnntg agccatgatc 410
<210> 126
   <211> 23
   <212> DNA
   <213> Homo sapiens
<400> 126
   gttattacaa tgtcaccctc att 23
<210> 127
   <211> 24
   <212> DNA
   <213> Homo sapiens
<400> 127
   acatctgtaa gagcttcaca aaca 24
<210> 128
   <211> 199
   <212> DNA
   <213> Homo sapiens
<220>
   <221> misc_feature
   <222> (8)..(8)
   <223> n = a, c, g, or t/u
<400> 128
   attccttngt tattacaatg tcaccctcat ttaaaaagtg gaaagataaa gaggaagcaa 60
   tctatttttt tccttttttt ctgatagcac ttgtttgtga agctcttaca gatgttctta 120
   agtaaaatca actcctccat tttttttgta gcaactacac atatttatca ataatagttc 180
   acaaatacat tttcaaatt 199
<210> 129
   <211> 21
   <212> DNA
   <213> Homo sapiens
<400> 129
   acgtttctca attgtttagt c 21
<210> 130
   <211> 20
   <212> DNA
   <213> Homo sapiens
<400> 130
   tgtcttggca ttatttttac 20
<210> 131
   <211> 247
   <212> DNA
   <213> Homo sapiens
<220>
   <221> misc_feature
   <222> (214)..(214)
   <223> n = a, c, g, or t/u
<400> 131
   agacaatggg agaaattgca ctgccctgag tcagaaatca gatctgttgc catacagctg 60
   ccgttatgtg atcatttgca agtcaacgtt tctcaattgt ttagtcattt gtaagacaaa 120
   aagactggtt ggatttcaga gaatttggaa tcctccttca ggtttaacaa gcaataaatg 180
   atactcttca gtgtaaaaat aatgccaaga catnatttga ctttaaatta aatccaaaca 240
   agatatc 247
<210> 132
   <211> 24
   <212> DNA
   <213> Homo sapiens
<400> 132
   ctatgctctg atctttgtta cttt 24
<210> 133
   <211> 21
   <212> DNA
   <213> Homo sapiens
<400> 133
   attaacggga aagaatggta t 21
<210> 134
   <211> 20
   <212> DNA
   <213> Homo sapiens
<400> 134
   aaggtatatt atttgtgtcg 20
<210> 135
   <211> 20
   <212> DNA
   <213> Homo sapiens
<400> 135
   aaacttttct taacctcata 20
<210> 136
   <211> 24
   <212> DNA
   <213> Homo sapiens
<400> 136
   tctacacata tgggaaagca ggaa 24
<210> 137
   <211> 21
   <212> DNA
   <213> Homo sapiens
<400> 137
   gctggtggtt ttggaggtag g 21
<210> 138
   <211> 21
   <212> DNA
   <213> Homo sapiens
<400> 138
   ataagagacc agaatgtgat a 21
<210> 139
   <211> 19
   <212> DNA
   <213> Homo sapiens
<400> 139
   tctttggagg agggtagtc 19
<210> 140
   <211> 24
   <212> DNA
   <213> Homo sapiens
<400> 140
   aaatctctta agctcatgct agtg 24
<210> 141
   <211> 18
   <212> DNA
   <213> Homo sapiens
<400> 141
   cctgcctacc agcctgtc 18
<210> 142
   <211> 111
   <212> DNA
   <213> Homo sapiens
<400> 142
   agtggagaga tagaaagaga ggaagatttt tttttttaaa tctcttaagc tcatgctagt 60
   gtaggtgctg gcaggtctga acactctgta ggacaggctg gtaggcagga a 111
<210> 143
   <211> 18
   <212> DNA
   <213> Homo sapiens
<400> 143
   tggggtgcgc tgtgttgt 18
<210> 144
   <211> 24
   <212> DNA
   <213> Homo sapiens
<400> 144
   gagatttcat gcattcctgt aaga 24
<210> 145
   <211> 21
   <212> DNA
   <213> Homo sapiens
<400> 145
   gaggcacagc ttaccagttc a 21
<210> 146
   <211> 21
   <212> DNA
   <213> Homo sapiens
<400> 146
   attcattttc tcattttatc c 21
<210> 147
   <211> 18
   <212> DNA
   <213> Homo sapiens
<400> 147
   caagattatg cctcaact 18
<210> 148
   <211> 19
   <212> DNA
   <213> Homo sapiens
<400> 148
   taagctcata atctctgga 19
<210> 149
   <211> 209
   <212> DNA
   <213> Homo sapiens
<400> 149
   aaactttaac caatttaaac tccctaacag ttctataaaa taagcaagat tatgcctcaa 60
   ctttatggat aaagaaatgg aggcattaag agataactaa cttgcccaag gccacacaag 120
   tgactgagta agaattgcaa agccaatgag tctggctcca gagattatga gcttaatcac 180
   cacactgtgc cacctcctgt gtttcctgg 209
<210> 150
   <211> 23
   <212> DNA
   <213> Homo sapiens
<400> 150
   tcacttggtt ggttaacatt act 23
<210> 151
   <211> 24
   <212> DNA
   <213> Homo sapiens
<400> 151
   tagaaaaaca gttgcatttg atat 24
<210> 152
   <211> 20
   <212> DNA
   <213> Homo sapiens
<400> 152
   aacccaaggg agcacaactg 20
<210> 153
   <211> 20
   <212> DNA
   <213> Homo sapiens
<400> 153
   ggcaataggc tttccaacat 20
<210> 154
   <211> 17
   <212> DNA
   <213> Homo sapiens
<400> 154
   ttgtggaatg gctaagt 17
<210> 155
   <211> 18
   <212> DNA
   <213> Homo sapiens
<400> 155
   gaaagtatca aggcagtg 18
<210> 156
   <211> 22
   <212> DNA
   <213> Homo sapiens
<400> 156
   tatttggttt gtttgctgag gt 22
<210> 157
   <211> 23
   <212> DNA
   <213> Homo sapiens
<400> 157
   caagaaggat ggatacaaac aag 23
<210> 158
   <211> 17
   <212> DNA
   <213> Homo sapiens
<400> 158
   cgctatgcat ggattta 17
<210> 159
   <211> 18
   <212> DNA
   <213> Homo sapiens
<400> 159
   gctgaattta ggatgtaa 18
<210> 160
   <211> 91
   <212> DNA
   <213> Homo sapiens
<400> 160
   cgctatgcat ggatttaaac tgagtgtagt gcactcacta tgttgcagtc tcttattcta 60
   ggttcctaat atttacatcc taaattcagc t 91
<210> 161
   <211> 21
   <212> DNA
   <213> Homo sapiens
<400> 161
   ttcggatccg gtaccatcga t 21
<210> 162
   <211> 21
   <212> DNA
   <213> Homo sapiens
<400> 162
   ttatcgatgg taccggatcc g 21

## Description

The invention is concerned with the determination of genetic factors associated with psychiatric health with particular reference to a human gene or genes which contributes to or is responsible for the manifestation of a bipolar disorder in affected individuals. In particular, although not exclusively, the invention provides a method of identifying and characterising such a gene or genes from human chromosome 18. The invention is also concerned with methods of determining the genetic susceptibility of an individual to a bipolar disorder By bipolar disorders is meant the following disorders as defined in the Diagnostic and Statistical Manual of Mental Disorders, version 4 (DSM-IV) taxonomy (DSM-IV codes in parenthesis):- mood disorders (296.XX, 300.4, 311, 301.13, 295.70), schizophrenia and related disorders (295.XX, 297.1,298.8, 297.3, 298.9), anxiety disorders (300.XX, 309.81,308.3), adjustment disorders (309.XX) and personality disorders (codes 301.XX).

The methods of the invention are particularly exemplified in relation to genetic factors associated with a family of mood disorders known as Bipolar (BP) spectrum disorders.

Bipolar disorder (BP) is a severe psychiatric condition that is characterized by disturbances in mood, ranging from an extreme state of elation (mania) to a severe state of dysphoria (depression). Two types of bipolar illness have been described: type I BP illness (BPI) is characterized by major depressive episodes alternated with phases of mania, and type II BP illness (BPII), characterized by major depressive episodes alternating with phases of hypomania. Relatives of BP probands have an increased risk for BP, unipolar disorder (patients only experiencing depressive episodes; UP), cyclothymia (minor depression and hypomania episodes; CY) as well as for schizoaffective disorders of the manic (SAm) and depressive (SAd) type. Based on these observations BP, CY, UP and SA are classified as BP spectrum disorders. The involvement of genetic factors in the etiology of BP spectrum disorders was suggested by family, twin and adoption studies (Tsuang and Faraone (1990), The Genetics of Mood Disorders, Baltimore, The John Hopkins University Press). However, the exact pattern of transmission is unknown. In some studies, complex segregation analysis supports the existence of a single major locus for BP (Spence e*t al*. (1995), Am J. Med. Genet (Neuropsych. Genet.) 60 pp 370-376). Other researchers propose a liability-threshold-model, in which the liability to develop the disorder results from the additive combination of multiple genetic and environmental effects (McGuffin et al. (1994), Affective Disorders; Seminars in Psychiatric Genetics Gaskell, London pp 110-127).

Due to the complex mode of inheritance, parametric and nonparametric linkage strategies are applied in families in which BP disorder appears to be transmitted in a Mendelian fashion. Early linkage findings on chromosomes 11p15 (Egeland *et al.* (1987), Nature 325 pp 783-787) and Xq27-q28 (Mendlewicz *et al.* (1987) The Lancet 1 pp 1230 -1232; Baron et *al.* (1987) Nature 326 pp 289-292) have been controversial and could initially not be replicated (Kelsoe et al. (1989) Nature 242 pp 238-243; Baron et al. (1993) Nature Genet 3 pp 49-55). With the development of a human genetic map saturated with highly polymorphic markers and the continuous development of data analysis techniques, numerous new linkage searches were started. In several studies, evidence or suggestive evidence for linkage to particular regions on chromosomes 4, 12, 18, 21 and X was found (Blackwood et al. (1996) Nature Genetics 12 pp 427-430, Craddock et al. (1994) Brit J. Psychiatry 164 pp 355-358, Berrettini et al. (1994), Proc Natl Acad Sci USA 91 pp 5918-5921, Straub et al. (1994) Nature Genetics 8 pp 291-296 and Pekkarinen et al. (1995) Genome Research 5 pp 105-115). In order to test the validity of the reported linkage results, these findings have to be replicated in other, independent studies.

Recently, linkage of bipolar disorder to the pericentromeric region on chromosome 18 was reported (Berrettini *et al*. 1994). Also a ring chromosome 18 with break-points and deleted regions at 18pter-p11 and 18q23-qter was reported in three unrelated patients with BP illness or related syndromes (Craddock et al. 1994). The chromosome 18p linkage was replicated by Stine et al. (1995) Am J Hum Genet 57 pp 1384-1394, who also reported suggestive evidence for a locus on 18q21.2-q21.32 in the same study. Interestingly, Stine et al. observed a parent-of-origin effect: the evidence of linkage was the strongest in the paternal pedigrees, in which the proband's father or one of the proband's father's sibs is affected.

In an independent replication study, the present inventors tested linkage with chromosome 18 markers in 10 Belgian families with a bipolar proband. To localize causative genes the linkage analysis or likelihood method was used in these families. This method studies within a family the segregation of a defined disease phenotype with that of polymorphic genetic markers distributed in the human genome. The likelihood ratio of observing cosegregation of the disease and a genetic marker under linkage versus no linkage is calculated and the log of this ratio or the log of the odds is the LOD score statistic z. A LOD score of 3 (or likelihood ratio of 1000 or greater) is taken as significant statistical evidence for linkage. In the inventors' study no evidence for linkage to the pericentromeric regions was found, but in one of the families, MAD31, a Belgian family of a BPII proband, suggestive linkage was found with markers located at 18q21.33-q23 (De bruyn et al. (1996) Biol Psychiatry 39 pp 679-688). Multipoint linkage analysis gave the highest LOD score in the interval between STR (Short Tandem Repeats) polymorphisms D18S51 and D18S61, with a maximum multipoint LOD score of +1.34. Simulation studies indicated that this LOD score is within the range of what can be expected for a linked marker given the information available in the family. Likewise, an affected sib-pair analysis also rejected the null-hypothesis of nonlinkage for several of the markers tested. Two other groups also found evidence for linkage of bipolar disorder to 18q (Freimer et al. (1996) Nature Genetics 12 pp 436-441, Coon et al. (1996) Biol Psychiatry 39 pp 689 to 696). Although the candidate regions in the different studies do not entirely overlap, they all suggest the presence of a susceptibility locus at 18q21-q23.

The inventors have now carried out further investigations into the 18q chromosomal region in family MAD31. By analysis of cosegregation of bipolar disease in MAD31 with twelve STR polymorphic markers previously located between the aforementioned markers D18S51 and D18S61 and subsequent LOD score analysis as described above, the inventors have further refined the candidate region of chromosome 18 in which a gene associated with mood disorders such as bipolar spectrum disorders may be located and have constructed a physical map. The region in question may thus be used to locate, isolate and sequence a gene or genes which influences psychiatric health and mood.

The inventors have also constructed a YAC (yeast artificial chromosome) contig map of the candidate region to determine the relative order of the twelve STR markers mapped by the cosegregational analysis and they have identified seven clones from the YAC library incorporating the candidate region.

A number of procedures can be applied to the identified YAC clones and, where applicable, to the DNA of an individual afflicted with a bipolar disorder as defined herein, in the process of identifying and characterising the relevant gene or genes. For example, the inventors have used YAC clones spanning the region of interest in chromosome 18 to identify by CAG or CTG fragmentation novel genes that are allegedly involved in the manifestation of mood disorders or related disorders.

Other procedures can also be applied to the said YAC clones to identify candidate genes as discussed below.
Once candidate genes have been identified it is possible to assess the susceptibility of an individual to a bipolar disorder by detecting the presence of a polymorphism associated with a bipolar disorder in such genes.

Accordingly, in a first aspect the present invention comprises the use of an 8.9 cM region of human chromosome 18q disposed between polymorphic markers D18S68 and D18S979 or a fragment thereof for identifying at least one human gene, including mutated and polymorphic variants thereof, which contributes to the manifestation of bipolar disorder as defined above. As will be described below, the present inventors have identified this candidate region of chromosome 18q for such a gene, by analysis of cosegregation of bipolar disease in family MAD31 with 12 STR polymorphic markers previously located between D18S51 and D18S61 and subsequent LOD score analysis.

In a second aspect the invention comprises the use of a YAC clone comprising a portion of human chromosome 18q disposed between polymorphic markers D18568 and D185979 for identifying at least one human gene, including mutated or polymorphic variants thereof, which is associated with mood disorders or related disorders as defined above.

Particular YACs covering the candidate region which may be used in accordance with the present invention are 961_h_9, 942_c_3, 766_f_12, 731_c_7, 907_e_1, 752_g_8 and 717_d_3, preferred ones being 961. h_9, 766_f_2 and 907_e_1 since these have the minimum tiling path across the candidate region. Suitable YAC clones for use are those having an artificial chromosome spanning the refined candidate region between D18S68 and D18S979.

There are a number of methods which can be applied to the candidate regions of chromosome 18q as defined above, whether or not present in a YAC, to identify a candidate gene or genes associated with mood disorders or related disorders. For example, it has previously been demonstrated that an apparent association exists between the presence of trinucleotide repeat expansions (TRE) in the human genome and the phenomenon of anticipation of mood disorders (Lindblad et al. (1995), Neurobiology of Disease 2: 55-62 and O'Donovan et al. (1995), Nature Genetics 10: 380-381).

Accordingly, in a third aspect the present invention comprises a method of identifying at least one human gene, including mutated and polymorphic variants thereof, which is associated with a mood disorder or related disorder as defined herein which comprises detecting nucleotide triplet repeats in the region of human chromosome 18q disposed between polymorphic markers D18S68 and D18S979.

An alternative method of identifying said gene or genes comprises fragmenting a YAC clone comprising a portion of human chromosome 18q disposed between polymorphic markers D18368 and D185979 for example one or more of the seven aforementioned YAC clones, and detecting any nucleotide triplet repeats in said fragments. Nucleic acid probes comprising at least 5 and preferably at least 10 CTG and/or CAG triplet repeats are a suitable means of detection when appropriately labelled. Trinucleotide repeats may also be determined using the known RED (repeat expansion detection) system (Shalling et al.(1993), Nature Genetics 4 pp 135-139).

As will be described in detail below, in order to identify candidate genes containing triplet repeats, the inventors have carried out direct CAG or CTG fragmentation of YACs 961_h_9, 766_f_12 and 907_e_1, comprising a portion of human chromosome 18q disposed between polymorphic markers D18S60 and D18S61, and have identified a number of sequences containing CAG or CTG repeats, whose abnormal expansion may be involved in genetic susceptibility to a mood disorder or related disorder. These nucleotide sequences are shown in Figures 15a, 16a, 17a, and 18a.

Knowledge of the sequences described above can be used to design assays to determine the genetic susceptibility of an individual to a biopolar disorder.

Accordingly, in a further aspect the invention provides a method for determining the susceptibility of an individual to a bipolar disorder which comprises the steps of:
a) obtaining a DNA sample from said individual;
b) providing primers suitable for the amplification of a nucleotide sequence comprised in the sequence shown in any one of Figures 15a, 16a, 17a or 18a said primers flanking the trinucleotide repeats comprised in said sequence;
c) applying said primers to the said DNA sample and carrying out an amplification reaction;
d) carrying out the same amplification reaction on a DNA sample from a control individual; and
e) comparing the results of the amplification reaction for the said individual and for the said control individual;
wherein the presence of an amplified fragment from said individual which is bigger in size from that of said control individual is an indication of the presence of a susceptibility to a bipolar disorder of said individual.
By control individual is meant an individual who is not affected by a bipolar disorder and does not have a family history of mood disorders or related disorders.
Preferable primers to use in this method are those shown in Figure 15b, 16b, 17b or 18b but other suitable primers may be utilised.

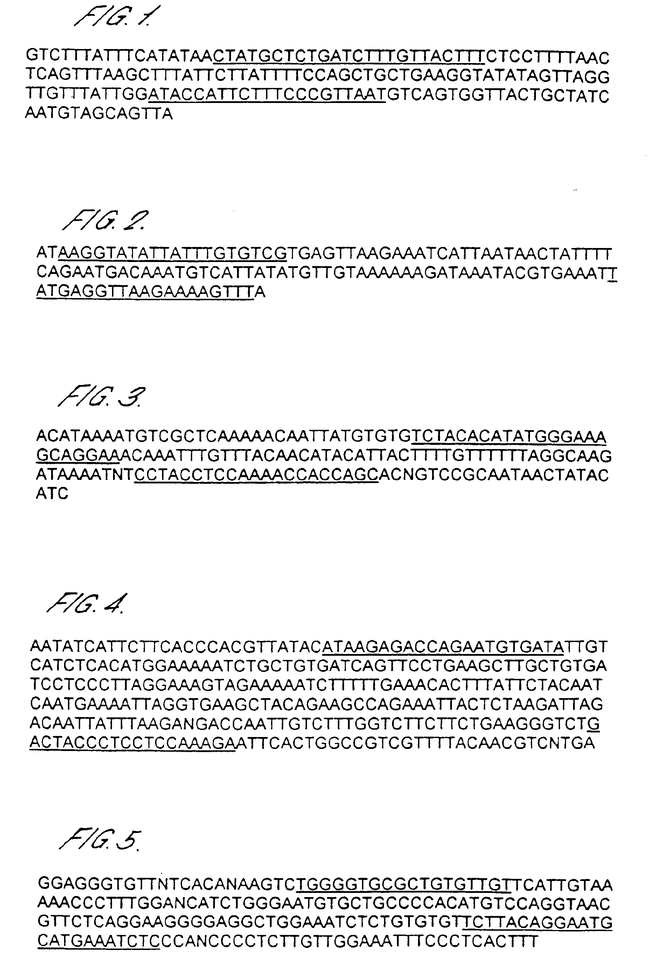

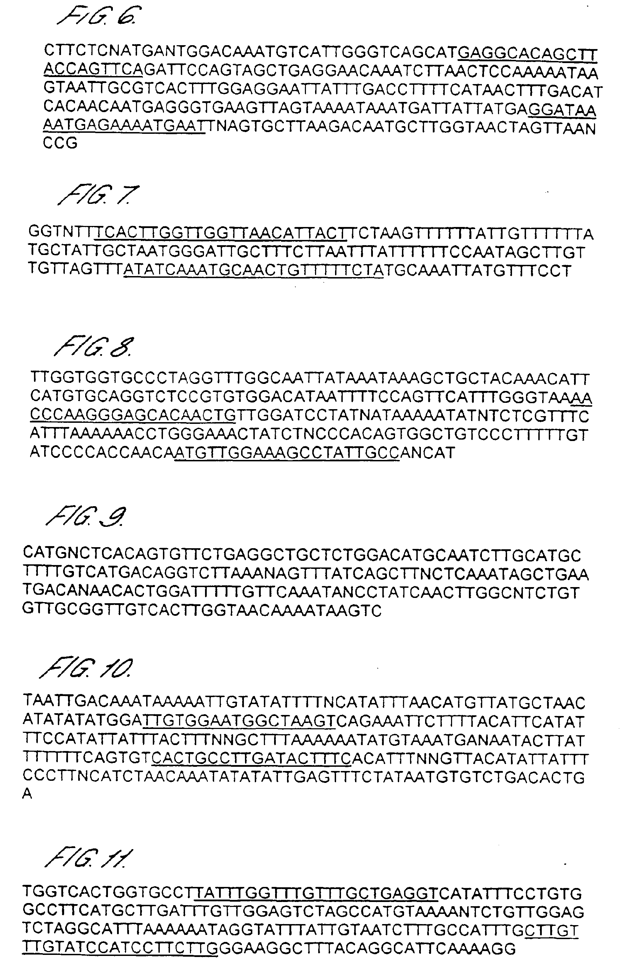

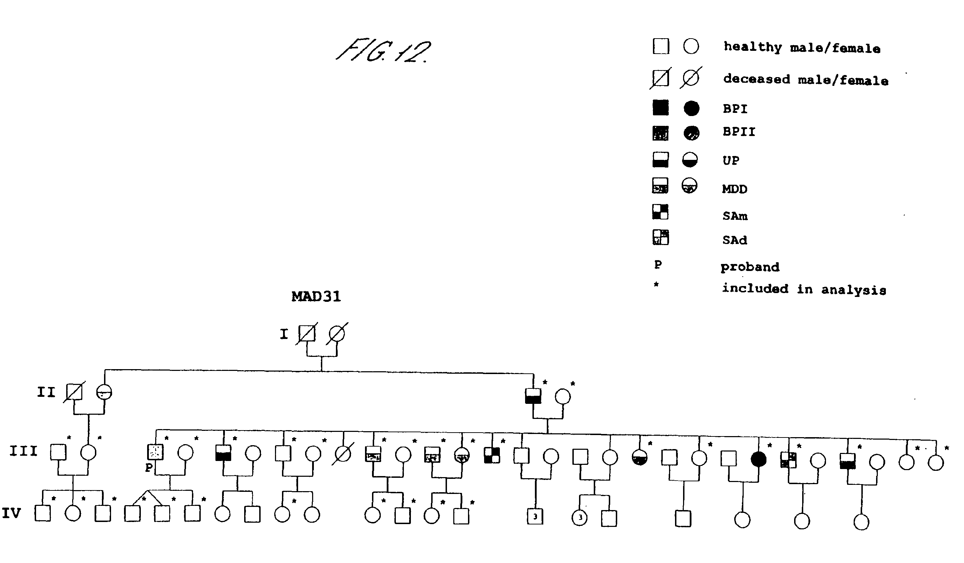

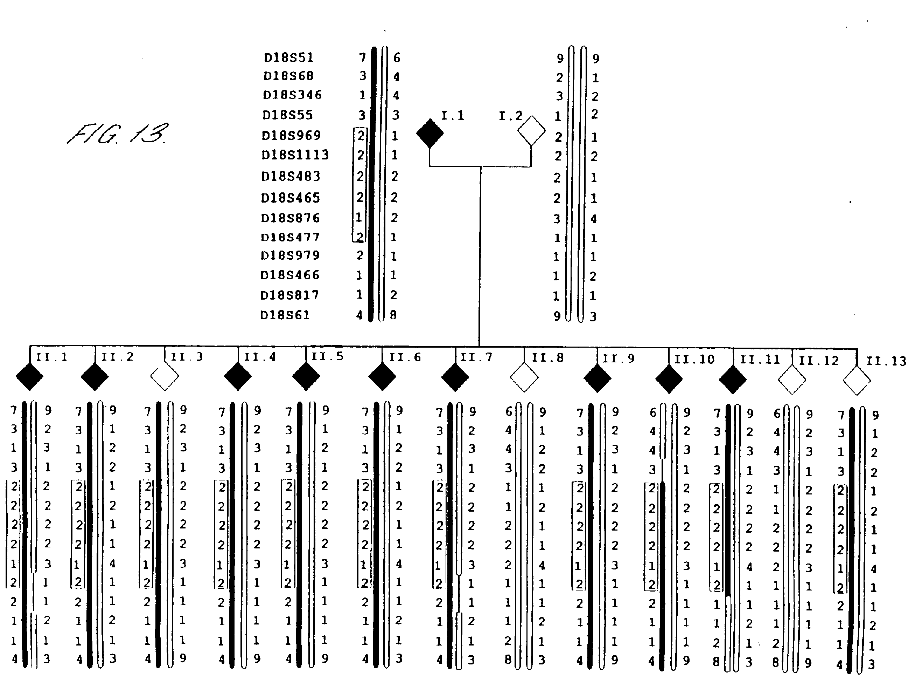

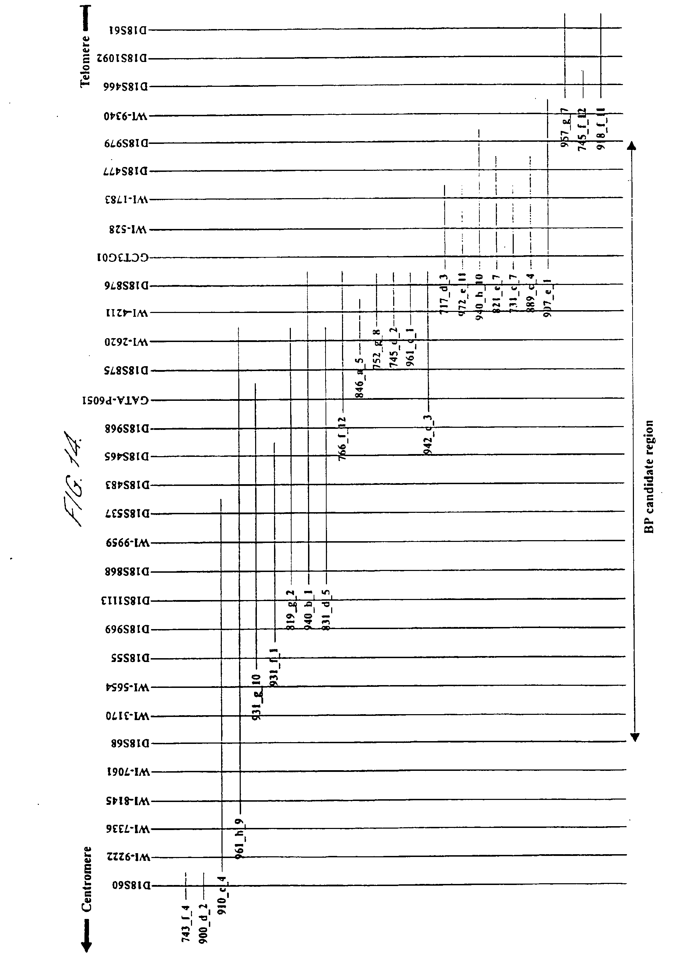

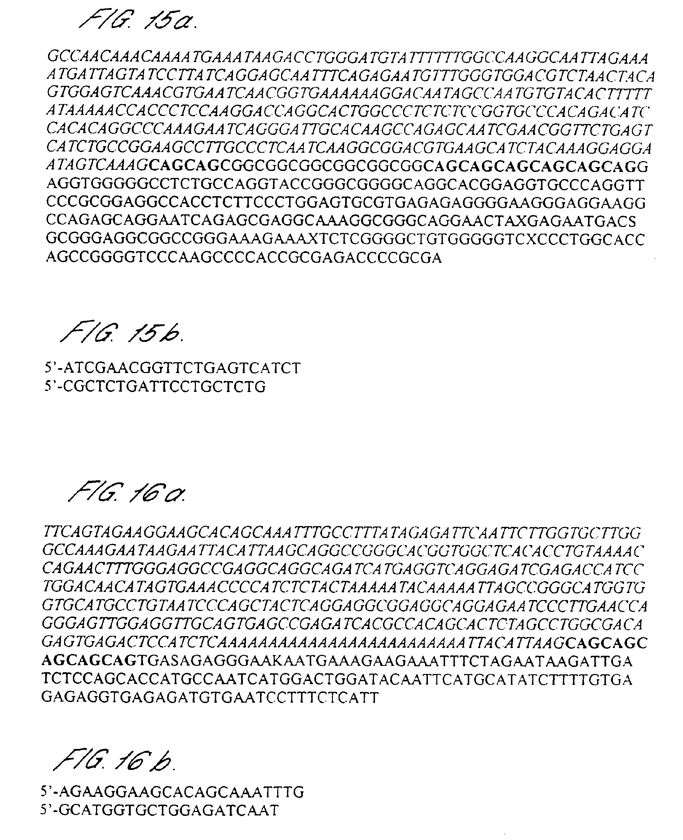

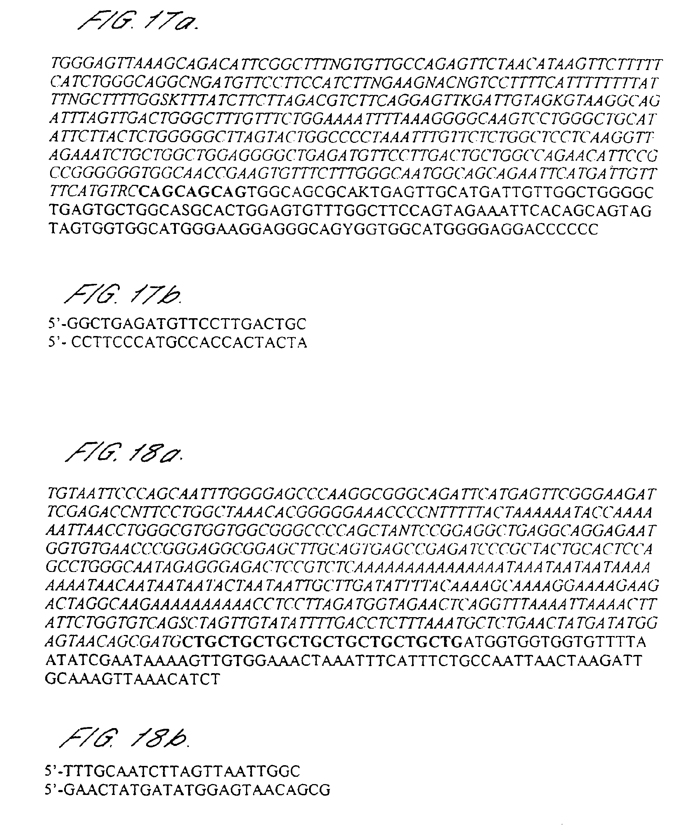

Further embodiments of the present invention relate to methods of identifying the relevant gene or genes which involve the sub-cloning of YAC DNA as defined above into vectors such as BAC (bacterial artificial chromosome) or PAC (P1 or phage artificial chromosome) or cosmid vectors such as exon-trap cosmid vectors. The starting point for such methods is the construction of a contig map of the region of human chromosome 18q between polymorphic markers D18568 and D185979. To this end the present inventors have sequenced the end regions of the fragment of human DNA in each of the seven aforementioned YAC clones and these sequences are disclosed herein. Following subcloning of YAC DNA into other vectors as described above, probes comprising these end sequences or portions thereof, in particular those sequences shown in Figures 1 to 11 herein, together with any known sequenced tagged site (STS) in this region, as described in the YAC clone contig shown herein, as can be used to detect overlaps between said subclones and a contig map can be constructed. Also the known sequences in the current YAC contig can be used for the generation of contig map subclones.

One route by which a gene or genes which is associated with a bipolar disorder can be identified is by use of the known technique of exon trapping.

This is an artificial RNA splicing assay, most often making use in current protocols of a specialized exon-trap cosmid vector. The Vector contains an artificial minigene consisting of a segment of the SV40 genome containing an origin of replication and a powerful promoter sequence, two splicihg-competent exons separated by an intron which contains a multiple cloning site and an SV40 polyadenylation site.

The YAC DNA is subcloned in the exon-trap vector and the recombinant DNA is transfected into a strain of mammalian cells. Transcription from the SV40 promoter results in an RNA transcript which normally splices to include the two exons of the minigene. If the cloned DNA itself contains a functional exon, it can be spliced to the exons present in the vector's minigene. Using reverse transcriptase a cDNA copy can be made and using specific PCR primers, splicing events involving exons of the insert DNA can be identified. Such a procedure can identify coding regions in the YAC DNA which can be compared to the equivalent regions of DNA from a person afflicted with a mood disorder or related disorder to identify the relevant gene.

Accordingly, in a further aspect the invention comprises a method of identifying at least one human gene, including mutated variants and polymorphisms thereof, which is associated with a bipolar disorder which comprises the steps of:
(a) transfecting mammalian cells with exon trap cosmid vectors prepared and mapped as described above;
(b) culturing said mammalian cells in an appropriate medium;
(c) isolating RNA transcripts expressed from the SV40 promoter;
(d) -preparing cDNA from said RNA transcripts;
(e) identifying splicing events involving exons of the DNA subcloned into said exon trap cosmid vectors to elucidate positions of coding regions in said subcloned DNA;
(f) detecting differences between said coding regions and equivalent regions in the DNA of an individual afflicted with said bipolar disorder ; and
(g) identifying said gene or mutated or polymorphic variant thereof which is associated with said bipolar disorder

As an alternative to exon trapping the YAC DNA may be subcloned into BAC, PAC, cosmid or other vectors and a contig map constructed as described above. There are a variety of known methods available by which the position of relevant genes on the subcloned DNA can be established as follows:
(a) cDNA selection or capture (also called direct selection and cDNA selection): this method involves the forming of genomic DNA/cDNA heteroduplexes by hybridizing a cloned DNA (e.g. an insert of a YAC DNA), to a complex mixture of cDNAs, such as the inserts of all cDNA clones from a specific (e.g. brain) cDNA library. Related sequences will hybridize and can be enriched in subsequent steps using biotinstreptavidine capturing and PCR (or related techniques);
(b) hybridization to mRNA/cDNA: a genomic clone (e.g. the insert of a specific cosmid) can be hybridized to a Northern blot of mRNA from a panel of culture cell lines or against appropriate (e.g. brain) cDNA libraries. A positive signal can indicate the presence of a gene within the cloned fragment;
(c) CpG island identification: CpG or HTF islands are short (about 1 kb) hypomethylated GC-rich (> 60%) sequences which are often found at the 5' ends of genes. CpG islands often have restriction sites for several rare-cutter restriction enzymes. Clustering of rare-cutter restriction sites is indicative of a CpG island and therefore of a possible gene. CpG islands can be detected by hybridization of a DNA clone to Southern blots of genomic DNA digested with rare-cutting enzymes, or by island-rescue PCR (isolation of CpG islands from YACs by amplifying sequences between islands and neighbouring *Alu-*repeats);
(d) zoo-blotting: hybridizing a DNA clone (e.g. the insert of a specific cosmid) at reduced stringency against a Southern blot of genomic DNA samples from a variety of animal species. Detection of hybridization signals can suggest conserved sequences, indicating a possible gene.

Accordingly, in a further aspect the invention comprises a method of identifying at least one human gene including mutated and polymorphic variants thereof which is associated with a bipolar disorder which comprises the steps of:
(a) subcloning the YAC DNA as described above into a cosmid, BAC, PAC or other vector;
(b) using the nucleotide sequences shown in any one of Figures 1 to 11 or any other sequenced tagged site (STS) in this region as in the YAC clone contig described herein, or part thereof consisting of not less than 14 contiguous bases or the complement thereof, to detect overlaps amongst the subclones and construct a map thereof;
(c) identifying the position of genes within the subcloned DNA by one or more of CpG island identification, zoo-blotting, hybridization of the subcloned DNA to a cDNA library or a Northern blot of mRNA from a panel of culture cell lines;
(d) detecting differences between said genes and equivalent region of the DNA of an individual afflicted with a bipolar disorder; and
(e) identifying said gene which is associated with said bipolar disorders

If the cloned YAC DNA is sequenced, computer analysis can be used to establish the presence of relevant genes. Techniques such as homology searching and exon prediction may be applied.

Once a candidate gene has been isolated in accordance with the methods of the invention more detailed comparisons may be made between the gene from a normal individual and one afflicted with a bipolar spectrum disorder. For example, there are two methods, described as "mutation testing", by which a mutation or polymorphism in a DNA sequence can be identified. In the first the DNA sample may be tested for the presence or absence of one specific mutation but this requires knowledge of what the mutation might be. In the second a sample of DNA is screened for any deviation from a control (normal) DNA. This latter method is more useful for identifying candidate genes where a mutation is not identified in advance.

In addition, the following techniques may be further applied to a gene identified by the above-described methods to identify differences between genes from normal or healthy individuals and those afflicted with a bipolar disorder:
(a) Southern blotting techniques: a clone is hybridized to nylon membranes containing genomic DNA digested with different restriction enzymes of patients and healthy individuals. Large differences between patients and healthy individuals can be visualized using a radioactive labelling protocol;
(b) heteroduplex mobility in polyacrylamide gels: this technique is based on the fact that the mobility of heteroduplexes in non-denaturing polyacrylamide gels is less than the mobility of homoduplexes. It is most effective for fragments under 200 bp;
(c) single-strand conformational polymorphism analysis (SSCP or SSCA): single stranded DNA folds up to form complex structures that are stabilized by weak intramolecular bonds. The electrophoretic mobilities of these structures on non-denaturing polyacrylamide gels depends on their chain lengths and on their conformation;
(d) chemical cleavage of mismatches (CCM): a radiolabelled probe is hybridized to the test DNA, and mismatches detected by a series of chemical reactions that cleave one strand of the DNA at the site of the mismatch. This is a very sensitive method and can be applied to kilobase-length samples;
(e) enzymatic cleavage of mismatches: the assay is similar to CCM, but the cleavage is performed by certain bacteriophage enzymes.
(f) denaturing gradient gel electrophoresis: in this technique, DNA duplexes are forced to migrate through an electrophoretic gel in which there is a gradient of increasing amounts of a denaturant (chemical or temperature). Migration continues until the DNA duplexes reach a position on the gel wherein the strands melt and separate, after which the denatured DNA does not migrate much further. A single base pair difference between a normal and a mutant DNA duplex is sufficient to cause them to migrate to different positions in the gel;
(g) direct DNA sequencing.

It will be appreciated that with respect to the methods described herein, in the step of detecting differences between coding regions from the YAC and the DNA of an individual afflicted with a bipolar disorder, the said individual may be anybody with the disorder and not necessary a member of family MAD31.

In the experimental report which follows reference will be made to the following figures:
FIGURE 1 shows a sequence of nucleotides which is the left arm end-sequence of YAC -7.66.f.12;
FIGURE 2 shows a sequence of nucleotides which is a right arm end-sequence of YAC 766_f_12;
FIGURE 3 shows a sequence of nucleotides which is a left arm end-sequence of YAC 717_d_3;
FIGURE 4 shows a sequence of nucleotides which is a right arm end-sequence of YAC 717_d_3;
FIGURE 5 shows a sequence of nucleotides which is a right arm end-sequence of YAC 731_c_7;
FIGURE 6 shows a sequence of nucleotides which is a left arm end-sequence of YAC 752_g_8;
FIGURE 7 shows a sequence of nucleotides which is a left arm end-sequence of YAC 942_c_3;
FIGURE 8 shows a sequence of nucleotides which is a right arm end-sequence of YAC 942_c_3;
FIGURE 9 shows a sequence of nucleotides which is a left arm end-sequence of YAC 961_h_9;
FIGURE 10 shows a sequence of nucleotides which is a right arm end-sequence of YAC 961_h_9;
FIGURE 11 shows a sequence of nucleotides which is a left arm end-sequence of YAC 907_e_1;
FIGURE 12 shows a pedigree of family MAD31;
FIGURE 13 shows the haplotype analysis for family MAD13. Affected individuals are represented by filled diamonds, open diamonds represent individuals who were asymptomatic at the last psychiatric evaluation. Dark gray bars represent markers for which it cannot be deduced if they are recombinant; and
FIGURE 14 shows the YAC contig map of the region of human chromosome 18 between the polymorphic markers D18560 and D18561. Black lines represent positive hits. YACs are not drawn to scale.
FIGURE 15 shows (a) a CAG repeat (in bold) and surrounding nucleotide sequence isolated from YAC 961_h_9. The sequence in italics is derived from End Rescue of the fragmented YAC. (b) PCR primers that can be used to determine the extent of trinucleotide repeats in the sequence.
FIGURE 16 shows (a) a CAG repeat (in bold) and surrounding nucleotide sequence isolated from YAC 766_f_12. The sequence in italics is derived from End Rescue of the fragmented YAC. (b) PCR primers that can be used to determine the extent of trinucleotide repeats in the sequence.
FIGURE 17 shows (a) a CAG repeat (in bold) and surrounding nucleotide sequence isolated from YAC 766_f_12. The sequence in italics is derived from End Rescue of the fragmented YAC. (b) PCR primers that can be used to determine the extent of trinucleotide repeats in the sequence.
FIGURE 18 shows (a) a CTG repeat (in bold) and surrounding nucleotide sequence isolated from YAC 907_e_1. The sequence in italics is derived from End Rescue of the fragmented YAC. (b) PCR primers that can be used to determine the extent of trinucleotide repeats in the sequence.

### Experimental 1

### (a) Family Data

Clinical diagnoses in MAD31, a Belgian family with a BPII proband were described in detail in De bruyn et al 1996. In that study only the 15 family members who were informative for linkage analysis were selected for additional genotyping. The different clinical diagnoses in the family were as follows: 1 BPI, 2 BPII, 2UP, 4 Major depressive disorder (MDD), 1 SAm and 1 SAd.
The pedigree of the MAD31 family is shown in Figure 12.

### (b) Genotyping of Family Members

All short tandem repeat (STR) genetic markers are di- or tetranucleotide repeat polymorphisms. Information concerning the genetic markers used in this study was obtained from several sources on the internet: Genome DataBase (GDB, http://gdbwww.gdb.org/), GenBank (http://www.ncbi.nlm.nih.gov/), Cooperative Human Linkage Center (CHLC, http://www.chlc.org/), Eccles Institute of Human Genetics (EIHG, http://www.genetics.utah.edu/) and Généthon (http://www.genethon.fr/). Standard PCR was performed in a 25 µl volume containing 100 ng genomic DNA, 200 mM of each dNTP, 1.25 mM MgCl₂, 30 pmol of each primer and 0.2 units Goldstar DNA polymerase (Eurogentec). One primer was end-labelled before PCR with [gamma-³²P]ATP and T4 polynucleotide kinase. After an initial denaturation step at 94°C for 2 min, 27 cycles were performed at 94°C for 1 min, at the appropriate annealing temperature for 1.5 min and extension at 72°C for 2 min. Finally, an additional elongation step was performed at 72°C for 5 min. PCR products were detected by electrophoresis on a 6% denaturing polyacrylamide gel and by exposure to an X-ray sensitive film. Successfully analysed STSs, STRs and ESTs covering the refined candidate region are fully described herein on pages 36 to 54.

### (c) Lod score analysis.

Two-point lod scores were calculated for 3 different disease models using Fastlink 2.2. (Cottingham et al. 1993). For all models, a disease gene frequency of 1% and a phenocopy rate of 1/1000 was used. Model 1 included all patients and unaffected individuals with the latter individuals being assigned to a disease penetrance class depending on their age at examination. The 9 age-dependent penetrance classes as described by De bruyn et al (1996) were multiplied by a factor 0.7 corresponding to a reduction of the maximal penetrance of 99% to 70% for individuals older than 60 years (Ott 1991). Model 2 is similar to model 1, but patients were assigned a diagnostic stability score, calculated based on clinical data such as the number of episodes, the number of symptoms during the worst episode and history of treatment (Rice et al. 1987, De bruyn et al. 1996). Model 3 is as model 1 but includes only patients.

### (d) Construction of the YAC contig - protocols

Growing of YACs and extraction of YAC DNA was done according to standard protocols (Silverman, 1995). For the construction of the YAC-contig spanning the chromosome 18q candidate region, the data of the physical map based on sequence tagged sites (STSs) (Hudson et al. 1995) was consulted on the Whitehead Institute (WI) Internet site (http://www-genome.wi.mit.edu/). CEPH mega-YACs were obtained from the YAC Screening Centre Leiden (YSCL, the Netherlands) and from CEPH (Paris, France). The YACs were analyzed for the presence of STSs and STRs, previously located between D18S51 and D18S61, by touchdown PCR amplification. Information on the STSs/STRs was obtained from the WI, GDB, Généthon, CHLC and GenBank sites on the Internet. Thirty PCR cycles consisted of: denaturation at 94°C for 1 min, annealing (2 cycles for each temperature) starting from 65°C and decreasing to 51°C for 1.5 min and extension at 72°C for 2 min. This was followed by 10 cycles of denaturation at 94°C for 1 min, annealing at 50°C for 1.5 min and extension at 72°C for 2 min. A final extension step was performed for 10 min at 72°C. Amplified products were visualised by electrophoresis on a 1% TBE agarose gel and ethidium bromide staining.

### (e) Ordering of the STR markers.

Twelve STR markers, previously located between D18S51 and D18S61, were tested for cosegregation with bipolar disease in family MAD31. The parental haplotypes were reconstructed from genotype information of the siblings in family MAD31 and minimalizing the number of possible recombinants. The result of this analysis is shown in Figure 13. The father was not informative for 3 markers, the mother was not informative for 5 markers. Haplotypes in family MAD31 suggested the following order for the STR markers analysed: cen-[S51-S68-S346]-[S55-S969-S1113-S483-S465]-[S876-S477]-S979-[S466-S817-S61]-tel. The order relative to each other of the markers between brackets could not be inferred from our haplotype data. The marker order in family MAD31 was compared with the marker order obtained using different mapping techniques and the results shown in Table 1 below.

**Table 1. Comparison of the order of the markers within the 18q candidate region for bipolar disorder, among several maps.**

| Marker* | Genetic maps | | Radiation hybrid map |
|---|---|---|---|
| | Généthon | Marshfield | (Giacalone et al. 1996) |
| D18S51 | | (-)3.4cM | (-)27.9 cR |
| D18568 | 0 cM | 0 cM | 0 cR |
| D185346 | | 5.3 cM | 52.2 cR |
| D18555 | 0.1 cM | 0 eM | 72.5 cR |
| D185969 | | 0.6 cM | |
| D18S 1113 | 0.7 cM | | |
| D185483 | 2.5 cM | 3.2 cM | 88 cR |
| D185465 | 4.5 cM | 5.3 cM | 101.3 cR |
| D18S876 | | | |
| D18S477 | 4.4 cM | 5.3 cM | 166.4 cR |
| D18S979 | | 8.9 cM | |
| D18S466 | 7.6 cM | 11.1 cm | 212.4 cR |
| D18S61 | 8.4 cM | 11.8 cm | 249.5 cR |
| D185817 | | 5.3 cM | 260.6 cR |

| | | | |
|---|---|---|---|
| * Order according to haplotyping results in family MAD31. | | | |
| (-) Marker is located proximal of D18568. | | | |

D18S68, common to all 3 maps, was taken as the map anchor point, and the genetic distance in cM or cR of the other markers relative to D18S68 are given. The marker order is in good agreement with the order of the markers on the recently published chromosome 18 radiation hybrid map (Giacalone et al. (1996) Genomics 37:9-18) and the WI YAC-contig map (http://www-genome.wi.mit.edu/). However, a few discrepancies with other maps were observed. The only discrepancy with the Généthon genetic map is the reversed order of D18S465 and D18S477. Two discrepancies were observed with the Marshfield map (http://www.marshmed.org/genetics/). The present inventors mapped D18S346 above D18S55 based on maternal haplotypes, but on the Marshfield maps D18S346 is located between D18S483 and D18S979. The inventors also placed D18S817 below D18S979, but on the Marshfield map this marker is located between D18S465 and D18S477. However, the location of D18S346 and D18S817 is in agreement with the chromosome 18 radiation hybrid map of Giacalone et al. (1996). One discrepancy was also observed with the WI radiation hybrid map (http://www-genome.wi.mit.edu/), in which D18S68 was located below D18S465. However, the inventors as well as other maps placed this marker above D18S55.

### (f) Lod score analysis and refinement of the candidate region.

Lod score analysis gave positive results with all markers, confirming the previous observation that 18q21.33-q23 is implicated in BP disease, at least in family MAD31 (De bruyn et al. 1996). Summary statistics of the lod score analysis under all models are given in table 2 below.

**Table 2. Summary statistics of the two-point lod scores in MAD31.**

| Marker | Model 1 | | | Model 2 | | | Model 3 | | |
|---|---|---|---|---|---|---|---|---|---|
| | Z at θ=0.0 | Zmax | θmax | Z at θ=0.0 | Zmax | θmax | Z at θ=0.0 | Zmax | θmax |
| D18S51 | -0.19 | 0.73 | 0.1 | 0.94 | 0.94 | 0.01 | 0.08 | 0.54 | 0.1 |
| D18S68 | -0.19 | 0.73 | 0.1 | 0.94 | 0.94 | 0.01 | 0.07 | 0.55 | 0.1 |
| D18S346 | -0.19 | 0.73 | 0.1 | 0.94 | 0.94 | 0.01 | 0.07 | 0.55 | 0.1 |
| D18969 | 1.40 | 1.40 | 0.0 | 1.27 | 1.27 | 0.0 | 1.20 | 1.20 | 0.0 |
| D18S1113 | 2.01 | 2.01 | 0.0 | 1.87 | 1.87 | 0.0 | 1.77 | 1.77 | 0.0 |
| D18S876 | 2.01 | 2.01 | 0.0 | 1.87 | 1.87 | 0.0 | 1.77 | 1.77 | 0.0 |
| D18S477 | 2.01 | 2.01 | 0.0 | 1.87 | 1.87 | 0.0 | 1.77 | 1.77 | 0.0 |
| D 185979 | -0.18 | 0.77 | 0.1 | 1.08 | 1.08 | 0.0 | 0.08 | 0.54 | 0.0 |
| D18S817 | -0.19 | 0.73 | 0.1 | 1.08 | 1.08 | 0.0 | 0.07 | 0.55 | 0.1 |
| D 18561 | -0.21 | 0.73 | 0.1 | 1.08 | 1.08 | 0.0 | 0.07 | 0.54 | 0.1 |

| | | | | | | | | | |
|---|---|---|---|---|---|---|---|---|---|
| D18S55, D18S483, D18S465 and D18S466 were not informative. | | | | | | | | | |

The highest two-point lod score (+2.01 at 8=0.0) was obtained with markers D18S1113, D18S876 and D18S477 under model 1 in the absence of recombinants (table 2). In model 1, all individuals with a BP spectrum disorder are considered affected and fully contributing to the linkage analysis. Before the fine mapping the candidate region was flanked by D18S51 and D18S61, which are separated by a genetic distance of 15.2 cM on the Marshfield map or 13.1 cM on the Généthon map. The informative recombinants with D18S51 and D18S61 were observed in 2 affected individuals (II.10 and II.11 in Fig. 13). However, since no other markers were tested within the candidate region it was not known whether these individuals actually shared a region identical-by-descent (IBD). The additional genetic mapping data now indicate that all affected individuals are sharing alleles at D18S969, D18S1113, D18S876 and D18S477 (Fig. 13, boxed haplotype). Also, alleles from markers D18S483 and D18S465 are probably IBD, but these markers were not informative in the affected parent 1.1. Obligate recombinants were observed with the STR markers D18S68, D18S346, D18S979 and D18S817 (Table 2, fig. 13) Since discrepancies between different maps were observed for the locations of D18S346 and D18S817, the present inventors used D18S68 and D18S979 to redefine the candidate region for BP disease. The genetic distance between these 2 markers is 8.9 cM based on the Marshfield genetic map (http.//www.marshmed.org/genetics/).

### (g) Construction of the YAC contig.

According to the WI integrated map 56 CEPH megaYACs are located in the initial candidate region contained between D18S51 and D18S61 (Chumakov et al. (1995) Nature 377 Suppl., De bruyn et al. (1996)). From these YACs, those were selected that were located in the region between D18S60 and D18S61. D18S51 is not presented on the WI map, but is located close to D18S60 according to the Marshfield genetic map (http.//www.marshmed.org/genetics/). To limit the number of potential chimaeric YACs, YACs were eliminated that were also positive for non-chromosome 18 STSs. As such, 25 YACs were selected (see Figure 14), and placed in a contig based on the technique of YAC contig mapping, i.e. sequences from sequence tagged sites (STSs), simple tandem repeats (STRs) and expressed sequenced tags (ESTs), known to map between D18S60 and D18S61, were amplified by PCR on the DNA from the YAC clones. The STS, STR and EST sequences used, are described from page 36 to 54. Positive YAC clones were assembled in a YAC contig map (Figure 14).

Three gaps remained in the YAC contig, of which one, between D18S876 and GCT3G01, was located in the refined candidate region. To close the gap between D18S876 and GCT3G01, 14 YAC clones (Table 3, on page 62) were further analysed. End fragments from YAC clones 766_f_12 (SV11R) , 752_g_8 (SV31L), 942_c_3 (SV10R) were obtained and sequenced (see pages 55-61). Primers from these three sequences were selected, and DNA of each of the 14 YAC clones was amplified by PCR. As indicated in Table 3, overlaps were obtained between 7 YAC clones on the centromeric side, and two YAC clones on the telomeric site (717_d_3 and 907_e_1).

The final YAC contig is shown in Figure 14. In the figure, only the YAC clones which rendered unambiguous hits with the chromosome 18 STSs, STRs and ESTs are shown. In a few cases, weak positive signals were also obtained with some of the YAC clones, which likely represent false positive results. However, these signals did not influence the alignment of the YAC clones in the contig. Although, all YACs known to map in the region were tested as well as all available STSs/STRs, initially, the gap in the YAC contig was not closed. However, this was subsequently achieved by determining the end-sequences of the eight selected YACs (see below). The order of the markers provided by the YAC contig map is in complete agreement with the marker order provided by the WI map which integrates information from the genetic map, the radiation hybrid map and the STS YAC contig map (Hudson et al. 1995). Also, the YAC contig map confirms the order of the STR markers as suggested by the haplotype analysis in family MAD31. Moreover, the YAC contig map provides additional information on the relative order of the STR markers. For example, D18S55 is present in YAC 931_g_10 but not in 931_f_1 (Fig.14), separating D18S55 from its cluster [S55-S969-S1113-S483-S465] obtained by haplotype analysis in family MAD31. The centromeric location of D18S55 is defined by the STS/STR content of surrounding YACs (Fig. 14). If we combine the haplotype data and the YAC contig map the following order of STR markers is obtained: cen-[S51-S68-S346)-S55-[S969-S113]-(S483-S465]-S876-S477-S979-S466-[S817-S61]-tel.

Out of the 25 YAC clones spanning the whole contig, seven YAC clones were selected in order to identify the minimal tiling path (Table 4). These 7 YAC clones cover the whole refined chromosome 18 region. Furthermore, YAC clones should preferably be non-chimeric, i.e. they should only contain fragments from human chromosome 18. In order to examine for the presence of chimerism, both ends of these YACs were subcloned and sequenced (pages 55 to 61). For each of the sequences, primers were obtained, and DNA from a monochromosomal mapping panel was amplified by PCR using these primers. As indicated on pages 55 to 61, some of the YAC clones contained fragments from other chromosomes, apart from human chromosome 18.

Three YAC clones were then selected comprising the minimum tiling path (Table 5). These three YAC clones were stable as determined by pulsed field gel electrophoresis and their seizes correspond well to the published sizes. These YAC clones were transferred to other host yeast strains for restriction mapping, and are the subject to further subcloning.

### Description of the succesfully analysed STSs, STRs and ESTs covering the refined candidate region.

### Explanations:

- STS: Sequence Tagged Site
- STR: Simple Tandem Repeat
- EST: Expressed Sequence Tag

These markers are ordered from the centromere to the telomere. Only the markers that were effectively tested and that worked on the YACs are given.

### List:

### 1. D18S60:

Database ID: AFM178XE3 (Also known as 178xe3, Z16781, D18S60)
Source: J Weissenbach, Genethon: genetically mapped polymorphic STSs
Chromosome: Chr18

### Primers:

Left = CCTGGCTCACCTGGCA
Right = TTGTAGCATCGTTGTAATGTTCC
Product Length = 157
Review complete sequence:

Genbank 10: Z16781
Description: H. sapiens (D18S60) DNA segment containing (CA) repeat; clone
Search for GDB entry

### 2. WI-9222:

Database ID: UTR-03540 (Also known as G06101, D18S1033, 9222, X63657)
Source: WICGR: Primers derived from Genbank sequences
Chromosome: Chr18

### Primers:

Left = GATCCCATAAAGCTACGAGGG
Right = GAGTCTAAAGACAAGAAAGCATTGC
Product Length = 99
Review complete sequence:

Genbank ID: X63657
Description: H.sapiens fvt1 mRNA
Search for GDB entry

### 3. WI-7336:

Database ID: UTR-04664 (Also known as P15, G00-679-135, G06527, 7336, U04313)
Source: WICGR: Primers derived from Genbank sequences
Chromosome: Chr18

### Primers:

Left = AGACATTCTCGCTTCCCTGA
Right = AATTTTGACCCCTTATGGGC
Product Length = 332
Review complete sequence:

Genbank ID: G06527
Description: WICGR: Random genome wide STSs

### 4. WI-8145:

Database ID: EST102441 (Also known as D18S1234, G00-677-827, G06845, 8145, T49159)
Source: WICGR: STSs derived from dbEST sequences
Chromosome: Chr18

### Primers:

Left = GAAATGCACATAACATATATTTGCC
Right = TGCTCACTGCCTATTTAATGTAGC
Product Length = 184
Review complete sequence:
: left and right primer

### PCR Conditions

Genbank ID: T49159
Description: yb09e07.s1 Homo sapiens cDNA clone 70692 3' similar to gb:J02685
UniGene Cluster Description: Human mRNA for Arg-Serpin (plasminogen activator-inhibitor 2, PAI-2) Search for GDB entry

### 5. WI-7061:

Database ID: UTR-02902 (Also known as PAl2, G00-678-979, G06377, 7061, M18082)
Source: WICGR: Primers derived from Genbank sequences
Chromosome: Chr18

### Primers:

Left = TGCTCTTCTGAACAACTTCTGC
Right = ATAGAAGGGCATGGAGGGAT
Product Length = 338
Review complete sequence:

### PCR Conditions

Genbank ID: G06377
Description: WICGR: Random genome wide STSs

### 6. D18S68:

Database ID: AFM243YB9 (Also known as 248yb9, Z17122, D18S68)
Source: J Weissenbach, Genethon: genetically mapped polymorphic STSs
Chromosome: Chr18

### Primers:

Left = ATGGGAGACGTAATACACCC
Right = ATGCTGCTGGTCTGAGG
Product Length = 285
Review complete sequence:

Genbank ID: Z17122
Description: H. sapiens (D18S68) DNA segment containing (CA) repeat; clone

### 7. WI-3170:

Database ID: MR3726 (Also known as D18S1037, G04207, HALd22f2, 3170)
Source: WICGR: Random genome wide STSs
Chromosome: Chr18

### Primers:

Left = TGTGCTACTGATTAAGGTAAAGGC
Right = TGCTTCTTCAATTTGTAGAGTTGG
Product Length = 156
Review complete sequence

Genbank ID: G04207
Description: WICGR: Random genome wide STSs

### 8. WI-5654:

Database ID: MR10908 (Also known as D18S1259, G00-678-695, G05278, 5654)
Source: WICGR: Random genome wide STSs
Chromosome: Chr18

### Primers:

Left = CTTAATGAAAACAATGCCAGAGC
Right = TGCAAAATGTGGAATAATCTGG
Product Length = 149
Review complete sequence:

Genbank ID: G05278
Description: WICGR: Random genome wide STSs

### 9. D18S55:

Database ID: AFM122XC1 (Also known as 122xc1, Z16621, D18S55, GC378-D18S55)
Source: J Weissenbach, Genethon: genetically mapped polymorphic STSs
Chromosome: Chr18

### Primers:

Left = GGGAAGTCAAATGCAAAATC
Right = AGCTTCTGAGTAATCTTATGCTGTG
Product Length = 143
Review complete sequence:

Genbank ID: Z16621
Description: H. sapiens (D18S55) DNA segment containing (CA) repeat; clone

### 10. D18S969:

Database ID: GATA-P18099 (Also known as G08003, CHLC.GATA69F01, CHLC.GATA69F01.P18099)
Source: CHLC: genetically mapped polymorphic tetranucleotide repeats
Chromosome: Chr18

### Primers:

Left = AACAAGTGTGTATGGGGGTG
Right = CATATTCACCCAGTTTGTTGC
Product Length = 365
Review complete sequence:

Genbank ID: G08003
Description: human STS CHLC.GATA69F01.P18099 clone GATA69F01.

### 11. D18S1113:

Database ID: AFM200VG9 (Also known as D18S1113, 200vg9, w2403)
Source: J Weissenbach, Genethon: genetically mapped polymorphic STSs
Chromosome: Chr18

### Primers:

Left = GTTGACTCAAGTCCAAACCTG
Right = CAAAGACATTGTAGACGTTCTCTG
Product Length = 207
Review complete sequence:

### 12. D1SS868:

Database ID: GATA-D18S868 (Also known as G09150, CHLC.GATA3E12, CHLC.GATA3E12.496. CHLC.496, D18S868)
Source: CHLC: genetically mapped polymorphic tetranucleotide repeats
Chromosome: Chr18

### Primers:

Left = AGCCAATACCTTGTAGTAAATATCC
Right = GATTCTCCAGACAAATAATCCC
Product Length = 189
Review complete sequence:

Genbank 10: G09150
Description: human STS CHLC.GATA3E12.P6553 clone GATA3E12.

### 13. WI-9959:

Database ID: MR12816 (Also known as D18S1251, G00-678-524, G05488, 9959)
Source: WICGR: Random genome wide STSs
Chromosome: Chr18

### Primers:

Left = TGCCAACAGCAGTCAAGC
Right = AGCACCTGCAGCAGTAATAGC
Product Length = 110
Review complete sequence:

Genbank ID: G05488
Description: WICGR: Random genome wide STSs
Search for GDB entry

### 14. D18S537:

Database ID: CHLC.GATA2E06.13 (Also known as CHLC.13, GATA2E06, D18S537, GATA-D18S537)
Source: CHLC: genetically mapped polymorphic tetranucleotide repeats
Chromosome: Chr18

### Primers:

Left = TCCATCTATCTTTGATGTATCTATG
Right = AGTTAGCAGACTATGTTAATCAGGA
Product Length = 191
Review complete sequence:

Genbank ID: G07990
Description: human STS CHLC.GATA2E06.P6006 clone GATA2E06.
Search for GDB entry

### 15. D18S483:

Database ID: AFM324WC9 (Also known as 324wc9, Z24399, D18S483)
Source: J Weissenbach, Genethon: genetically mapped polymorphic STSs
Chromosome: Chr18

### Primers:

Left = TTCTGCACAATTTCAATAGATTC
Right = GAACTGAGCAAACGAGTATGA
Product Length = 214
Review complete sequence:

Genbank ID: Z24399
Description: H. sapiens (D18S483) DNA segment containing (CA) repeat; clone
Search for GDB entry

### 16. D18S465:

Database ID: AFM250YH1 (Also known as 260yh1, Z23850, D18S465)
Source: J Weissenbach, Genethon: genetically mapped polymorphic STSs
Chromosome: Chr18

### Primers:

Left = ATATTCCCCTATGGAAGTACAG
Right = AAAGTTAATTTTCAGGCACTCT
Product Length = 232
Review complete sequence:

Genbank ID: Z23850
Description: H. sapiens (D18S465) DNA segment containing (CA) repeat; clone
Search for GDB entry

### 17. D18S968:

Database ID: GATA-P34272 (Also known as G10262, CHLC.GATA117C05, CHLC.GATA117C05.P34272)
Source: CHLC: genetically mapped polymorphic tetranucleotide repeats
Chromosome: Chr18

### Primers:

Left = GAAATTAACCAGACACTCCTAACC
Right = CTTAGAATTGCCTTTGCTGC
Product Length = 147
Review complete sequence:

Genbank ID: G10262
Description: human STS CHLC.GATA117C05.P34272 clone GATA117C05.

### 18. GATA-P6051:

Database ID: GATA-P6051 (Also known as CHLC.GATA3E08, CHLC.GATA3E08.P6051)
Source: CHLC: genetically mapped polymorphic tetranucleotide repeats
Chromosome: Chr18

### Primers:

Left = GCAACAACCCTAATGAGTATACG
Right = GAGTCTCACCAGGGCTTACA
Product Length = 149
Review complete sequence:

Genbank ID: G09104
Description: human STS CHLC.GATA3EO8.P6051 clone GATA3EO8.

### 19.D18S875:

Database ID: GATA-D18S875 (Also known as G08001, CHLC.GATA52HO4, D18S875)
Source: CHLC: genetically mapped polymorphic tetranucleotide repeats
Chromosome: Chr18

### Primers:

Left = TCCTCTCATCTCGGATATGG
Right = AAGGCTTTCAGACTTACACTGG
Product Length = 394
Review complete sequence:

Genbank ID: G08001
Description: human STS CHLC.GATA52H04.P16177 clone GATA52H04.
Search for GDB entry

### 20. WI-2620:

Database ID- MR1436 (Also known as G03602, D18S890, HHAa12h3, 2620)
Source: WICGR: Random genome wide STSs
Chromosome: Chr18

### Primers:

Left = TCTCCAAGCTATTGATTGGATAA
Right = TTAAGAGCCAATTTATATAAAAGCAGC
Product Length = 177
Review complete sequence:

Genbank ID: G03602
Description: WICGR: Random genome wide STSs
Search for GDB entry

### 21. WI-4211:

Database ID: MR6638 (Also known as G03617, D18S980, 4211)
Source: WICGR: Random genome wide STSs
Chromosome: Chr18

### Primers:

Left = ATGCTTCAGGATGACGTAATACA
Right = AAATTCTCGCTGATTGGAGG
Product Length = 113
Review complete sequence:

Genbank ID: G03617
Description: WICGR: Random genome wide STSs
Search for GDB entry

### 22. D18S876:

Database ID: GATA-D18S876 (Also known as G09963, CHLC.GATA61E10, D18S876)
Source: CHLC: genetically mapped polymorphic tetranucleotide repeats
Chromosome: Chr18

### Primers:

Left = TCAAACTTATAACTGCAGAGAACG
Right = ATGGTAAACCCTCCCCATTA
Product Length = 171
Review complete sequence:

Genbank ID: G09963
Description: human STS CHLC.GATA61E10.P17745 clone GATA61E10.
Search for GDB entry

### 23. GCT3G01:

Database ID: GCT-P10825 (Also known as G09484, CHLC.GCT3G01, CHLC.GCT3G01.P10825)
Source: CHLC: genetically mapped polymorphic tetranucleotide repeats
Chromosome: Chr18

### Primers:

Left = CTTTGCAATCTTAGTTAATTGGC
Right = GAACTATGATATGGAGTAACAGCG
Product Length = 128
Review complete sequence:

Genbank ID: G09484
Description: human STS CHLC.GCT3G01.P10825 clone GCT3G01.

### 24. WI-528:

Database ID: MH232 (Also known as G03589, 528, D18S828)
Source: WICGR: Random genome wide STSs
Chromosome: Chr18

### Primers:

Left = TTCTGCCTTTCCTGACTGTC
Right = TGTTTCCCATGTCTTGATGA
Product Length = 211
Review complete sequence:

Genbank ID: G03589
Description: WICGR: Random genome wide STSs
Search for GDB entry

### 25. WI-1783:

Database ID: MR432 (Also known as G03587, _shu_31.Seq, 1783, D18S824)
Source: WICGR: Random genome wide STSs
Chromosome: Chr18

### Primers:

Left = CCAGTAATTAGACATTGACAGGTTC
Right = TTTTACTAGACAGGCTTGATAAACAA
Product Length = 305
Review complete sequence:

Genbank ID: G03587
Description: WICGR: Random genome wide STSs
Search for GDB entry

### 26. D18S477:

Database ID: AFM301 XFS (Also known as 301xf5, Z24212, D18S477)
Source: J Weissenbach, Genethon: genetically mapped polymorphic STSs
Chromosome: Chr18

### Primers:

Left = GGACATCCTTGATTTGCTCATAA
Right = GATTGACTGAAAACAGGCACAT
Product Length = 243
Review complete sequence:

Genbank ID: Z24212
Description: H. sapiens (D18S477) DNA segment containing (CA) repeat; clone
Search for GDB entry

### 27. D18S979:

Database ID: GATA-P28080 (Also known as G08015, CHLC.GATA92C08, CHLC.GATA92C08. P28080)
Source: CHLC: genetically mapped polymorphic tetranucleotide repeats
Chromosome: Chr18

### Primers:

Left = AGCTTGCAGATAGCCTGCTA
Right = TACGGTAGGTAGGTAGATAGATTCG
Product Length = 155
Review complete sequence:

Genbank ID: G08015
Description: human STS CHLC.GATA92C08.P28080 clone GATA92C08.

### 28. WI-9340:

Database ID: UTR-05134 (Also known as G06102, D18S1034, 9340, X60221)
Source: WICGR: Primers derived from Genbank sequences
Chromosome: Chr18

### Primers:

Left = TGAGAGAACGAAATCTCTATCGG
Right = AGGCAGCAAGTTTTTATAAAGGC
Product Length = 115
Review complete sequence:

Genbank ID: G06102
Description: WICGR: Random genome wide STSs
Search for GDB entry

### 29. D18S466:

Database ID: AFMO94YE5 (Also known as 094ye5, Z23354, D18S466)
Source: J Weissenbach, Genethon: genetically mapped polymorphic STSs
Chromosome: Chr18

### Primers:

Left = ACACTGTAGCAGAGGCTTGACC
Right = AGGCCAAGTTATGTGCCACC
Product Length = 214
Review complete sequence:

Genbank ID: Z23354
Description: H. sapiens (D18S466) DNA segment containing (CA) repeat; clone
Search for GDB entry

### 30. D18S1092:

Database ID: AFMA112WE9 (Also known as D18S1092, w5374, a112we9)
Source: J Weissenbach, Genethon: genetically mapped polymorphic STSs
Chromosome: Chr18

### Primers:

Left = CTCTCAAAGTAAGAGCGATGTTGTA
Right = CCGAAGTAGAAAATCTTGGCA
Product Length = 163
Review complete sequence:
Search for GDB entry

### 31. D18S61:

Database ID: AFM193YF8 (Also known as 193yf8, Z16834, D18S61)
Source: J Weissenbach, Genethon: genetically mapped polymorphic STSs
Chromosome: Chr18

### Primers:

Left = ATTTCTAAGAGGACTCCCAAACT
Right = ATATTTTGAAACTCAGGAGCAT
Product Length = 174
Review complete sequence:

Genbank ID: Z16834
Description: H. sapiens (D18S61) DNA segment containing (CA) repeat; clone
Search for GDB entry

### Markers (STRs) used in refining the candidate region.

Below the markers are shown that were used in family MAD31 to refine the candidate region. Most of these markers are already described above and will therefore only be mentioned to by their name. For the additional markers, the information is given here.

Data was already shown for: D18S68, D18S55, D18S969, D18S1113, D18S483, D18S465, D18S876. D18S477, D18S979, D18S466 and D18S61.

### New data:

### 1. D18S51:

Other names: UT574, (D185379)
Primer sequences:
   UT574a GAGCCATGTTCATGCCACTG
   UT574b CAAACCCGACTACCAGCAAC

### DNA-sequence:

GENBANK ID: L18333

### 2. D18S346.

Other name: UT575
Primer Pairs:
   Primer A: TGGAGGTTGCAATGAGCTG
   Primer B: CATGCACACCTAATTGGCG

### DNA sequence:

GenBank ID: L26588

### 3. D18S817.

Other name: UT6365
Primer Pairs:
   Primer A: GCAAAGCAGAAGTGAGCATG
   Primer B: TAGGACTACAGGCGTGTGC

### DNA Sequence:

GenBank ID: L30552

### Characterisation of YACs.

8 YACs were selected covering the candidate region and flanking the gap. These YACs were further characterised by determining the end-sequences by the Inverse-PCR protocol.
Selected YACs: 961_h_9, 942_c_3, 766_f_12, 731_c_7, 907_e_1, 752_g_8, 717_ d_3, 745_d_2

New STSs based on end-sequences (unless indicated otherwise, the STSs were tested on a monochromosomal mapping pannel for identifying chimaerism of the YAC; if the STS revealed a hit not on chromosome 18q - chimaeric YAC- then it is indicated in the text below):

### 1. SV32L.

Derived from YAC 745_d_2 left arm end-sequence.
Primer A: GTTATTACAATGTCACCCTCATT
Primer B: ACATCTGTAAGAGCTTCACAAACA

### DNA-sequence:

Amplified sequence length: 107 basepairs (bp)

This STS has no clear hit on the monochromosomal mapping pannel.

### 2. SV32R.

Derived from YAC 745_d_2 right arm end-sequence.
Primer A: ACGTTTCTCAATTGTTTAGTC
Primer B: TGTCTTGGCATTATTTTTAC

### DNA sequence:

Amplified sequence length: 127 bp

This STS has no clear hit on the monochromosomal mapping pannel.

### 3. SV11L.

Derived from YAC 766_f_12 left arm end-sequence.
Primer A: CTATGCTCTGATCTTTGTTACTTT
Primer B: ATTAACGGGAAAGAATGGTAT

### DNA sequence:

Amplified sequence length: 118 bp

This STS has a hit with chromosome 18 and must ba located between CHLC.GATA-p6051 and D18S968.

### 4. SV11 R.

Derived from YAC 766_f_12 right arm end-sequence.
Primer A: AAGGTATATTATTTGTGTCG
Primer B: AAACTTTTCTTAACCTCATA

### DNA sequence:

Amplified sequence length: 119 bp. '

This STS has a hit with chromosome 18 and must be located between D18S876 and GCT3G01.

### 5. SV34L.

Derived from YAC 717_d_3 left arm end-sequence.
Primer A: TCTACACATATGGGAAAGCAGGAA
Primer B: GCTGGTGGTTTTGGAGGTAGG Amplified sequence length: 98 bp

This STS has a hit with chromosome 18.

### 6. SV34R.

Derived from YAC 717_d_3 right arm end-sequence.
Primer A: ATAAGAGACCAGAATGTGATA
Primer B: TCTTTGGAGGAGGGTAGTC

### DNA-sequence:

Amplified sequence length: 244 bp

This STS has a hit with chromosome 1, therefore YAC 717_d_3 is chimaeric

### 7. SV25L.

Derived from YAC 731_c_7 left arm end-sequence.
Primer A: AAATCTCTTAAGCTCATGCTAGTG
Primer B: CCTGCCTACCAGCCTGTC

### DNA sequence:

Amplified sequence length: 72 bp

This STS has no clear hits on the monochromosomal mapping pannel.

### 8. SV25R.

Derived from YAC 731_c_7 right arm end-sequence.
Primer A: TGGGGTGCGCTGTGTTGT
Primer B: GAGATTTCATGCATTCCTGTAAGA

### DNA-sequence:

Amplified sequence length: 136 bp

This STS has a hit with chromosome 7; therefore YAC 731_c_7 is chimaeric

### 9. SV31 L.

Derived from YAC 752_g_8 left arm end-sequence.
Primer A: GAGGCACAGCTTACCAGTTCA
Primer B: ATTCATTTTCTCATTTTATCC

### DNA-sequence:

Amplified sequence length: 178 bp

This STS has a hit with chromosome 18 and must be located between D18S876 and GCT3G01.

### 10. SV31 R.

Derived from YAC 752_g_8 right arm end-sequence.
Primer A: CAAGATTATGCCTCAACT
Primer B: TAAGCTCATAATCTCTGGA

### DNA sequence:

Amplified sequence length: 131 bp

This STS has no clear hits on the monochromosomal mapping pannel and gives no information concerning the chimaerity of the YAC.

### 11. SV10L.

Derived from YAC 942_c_3 left arm end-sequence.
Primer A: TCACTTGGTTGGTTAACATTACT
Primer B: TAGAAAAACAGTTGCATTTGATAT

### DNA-sequence:

Amplified sequence length: 130 bp

This STS has a hit with chromosome 18 and must be located between CHLC.GATA-p6051 and D18S968

### 12. SV10R.

Derived from YAC 942_c_3 right arm end-sequence.
Primer A: AACCCAAGGGAGCACAACTG
Primer B: GGCAATAGGCTTTCCAACAT

### DNA sequence:

Amplified sequence length: 135 bp

This STS has a hit with chromosome 18 and must be located between D18S876 and GCT3G01

### 13. SV6L.

Derived from YAC 961_h_9 left arm end-sequence.

No primer was made, because this sequence is identical to a known STR marker D18S42, which is indeed mapped to this region.
Primer A:
Primer B:

### DNA sequence:

Amplified sequence length:

SV6L recognises D18S42 which must be therefore located between WI-7336 and WI-8145

### 14. SV6R.

Derived from YAC 961_h_9 right arm end-sequence.
Primer A: TTGTGGAATGGCTAAGT
Primer B: GAAAGTATCAAGGCAGTG

### DNA sequence:

Amplified sequence length: 122 bp

SV6R amplifies a segment on chromosome 18. This segment must be located between WI-2620 and WI-4211

### 15. SV26L.

Derived from YAC 907_e_1 left arm end-sequence.
Primer A: TATTTGGTTTGTTTGCTGAGGT
Primer B: CAAGAAGGATGGATACAAACAAG

### DNA sequence:

Amplified sequence length: 154 bp

This STS has a hit with chromosome 13; therefore YAC 907_e_1 is chimaeric.

### 16. SV26R.

Derived from YAC 907_e_1 right arm end-sequence.
Primer A: CGCTATGCATGGATTTA
Primer B: GCTGAATTTAGGATGTAA

### DNA sequence:

Amplified sequence length: 90 bp
no clear hits on monochromosomal mapping pannel: no information concerning chaemerity at this side of the YAC

Testing of 3 end-sequences flanking the gap in additional YACs: STS-markers WI-4211, D18S876 and GCT3G01 are also shown in order to identify YACs on opposite sides of the gap more clearly in table 3 below.

| | STSs | | | | | |
|---|---|---|---|---|---|---|
| YACs | WI-4211 | D185876 | SV31 L | SV11R | SV10R | GCT3G01 |
| 940_b_1 | + | + | + | - | - | - |
| 766_f_12 | + | + | + | + | - | - |
| 846_a_5 | + | - ? | + | + | - | - |
| 752_g_ 8 | + | + | + | + | - | - |
| 745_d_2 | + | + | + | + | - | - |
| 961_c_1 | + | + | - | - | - | - |
| 942_c_3 | + | + | + | + | + | - |
| 717_d_3 | - | - | + | + | - ? | + |
| 972_e_11 | - | - | - | - | - | + |
| 940_h_10 | - | - | - | - | + | + |
| 821_e_7 | - | - | - | - | + | + |
| 731_c_7 | - | - | - | - | - | + |
| 889_c_4 | - | - | - | - | + | + |
| 907 e 1 | - | - | - | + | + | + |

| | | | | | | |
|---|---|---|---|---|---|---|
| • +: positive hit / -: no hit / ?: 2 instances were observed in which a positive hit was expected (on the assumed order of the markers) but not observed. The reasons for this are not clear. | | | | | | |

YAC 745.d.2 was excluded from further analysis since there was no clear hit with chromosome 18. Of the remaining 7 from a monochromosomal mapping panel it was determined that 3 were chimeric and 4 non-chimeric as shown in Table 4 below.

**TABLE 4**

| YAC | chimaeric | chromosome |
|---|---|---|
| 961_h_9 (6) | no | |
| 942_c_3 (10) | no | |
| 766_f_12 (11) | no | |
| 731_c_7 (25) | yes | chromosome 7 |
| 907_e_1 (26) | yes | chromosome 13 |
| 752_g_8 (31) | no | |
| 717_d_3 (34) | yes | chromosome 1 |

For the non-chimeric YACs the STS based on the end-sedquence flanking the gap (1 OR, 11 R, 31 L) was tested on 14 YACs flanking the gap. Overlaps between YACs on opposite sides of the gap were demonstrated: e.g. the "11 R" end-sequence (766_f_12) detects YAC 766_f_12 and YAC 907_e_1.
YACs were then selected comprising the minimum tiling path:

**TABLE 5**

| YAC | size | chimaerity |
|---|---|---|
| 961_h_9 | 1180 kb | not chimaeric |
| 766_f_12 | 1620 kb | not chimaeric |
| 907_e_1 | 1690 kb | chimaeric (chr. 13) |

These three YACs are stable as determined by PFGE and their sizes roughly correspond to the published sizes. These YACs were transferred to other host-yeast strains for restriction mapping.

### Experimental 2

### Construction of fragmentation vector:

A 4.5kb ECORI/SalI fragment of pBLC8.1 (Lewis et al, 1992) carrying a lysine-2 and a telomere sequence was directionally cloned into GEM3zf(-) digested with ECORI/SalI. Subsequently, an End Rescue Site was ligated into the EcoRI site. Hereto, two oligonucleotides (strand 1: 5'-TTCGGATCCGGTACCATCGAT-3' AND STRAND 2: 3'-GCCTAGGCCATGGTAGCTATT-5') were ligated into a partial (dATP) filled ECORI site, generating the vector pDF1. Triplet repeat containing fragmentation vectors were constructed by cloning of a 21bp and a 30bp CAG/CTG adapter into the Klenow-filled PstI site of pDF1. Trasformation and selection resulted in a (CAG)₇ and a (CTG)₁₀ fragmentation vector with the orientation of the repeat sequence 5' to 3' relative to the telomere.

### Yeast transformation:

Linearised (digested with SalI) vector was used to transform YAC clones 961.h.9, 766_f_12 or 907_e_1 using the LiAc method. After transformation the YAC clones were plated onto SDLys⁻ plates to select for the presence of the fragmentatio vector. After 2-3 days colonies were replica plated onto SDLys⁻-Trp⁻-Ura⁻ and SDLys⁻ -Trp⁻ -Ura⁺ plates. Colonies growing on the SDLys⁻-Trp⁻-Ura⁺ plates but not on the SDLys⁻-Trp⁻-Ura⁻ plates contained the fragmented YACs.

### Analysis of fragmented YACs:

Yeast DNA isolated from clones with the correct phenotype was analysed by Pulsed Field Electrophoresis (PFGE), followed by blotting and hybridisation with the Lys-2 gene and the sizes of the fragmented YACs were estimated by comparison with DNA standards of known length.

### End Rescue:

Fragmented YACs characterised by a size common to other fragmented YACs, indicative of the presence of a major CAG or CTG triplet repeat, were digested with one of the enzymes from the End Rescue site, ligated and used to transform E. Coli. After growth of the transformed bacteria the plasmid DNA was isolated and the ends of the fragmented YACs, corresponding to one of the sequences flanking the isolated trinucleotide repeats, were sequenced.

Sequencing revealed that fragmented YACs of an equal length were all fragmented at the same site. A BLAST Search of the GenBank database was performed with the identified sequences to identify homology with known sequences. The complete sequence spanning the CAG or CTG repeats of the fragmented YACs was obtained by Cosmid Sequencing, employing sequence specific primers and splice primers, as previously described (Fuentes et al. 1992 Hum.Genet. 101: 346-350) or by using the "genome walker" kit (Clontech Laboratories, Palo Alto, USA) and described in Siebert *et al.* Nucleic Acid Res (1995) 23(6): 1087-1088 and Siebert *et al.* (1995) CLONTECHniques X(II): 1-3.

### Results:

A YAC 961.h.9 clone was transformed with the (CAG)₇ or (CTG)₁₀ fragmentation vector. The CTG vector did not reveal the presence of any CTG repeat. Analysis of twelve (CAG)₇ fragmented YACs showed that five of these had the same size of approximately 100kb. End Rescue was performed with ECORI and sequencing of three of these fragments revealed that they all shared the terminal sequence shown in italics in Figure 15a. A BLAST search of the Genbank database with this sequence indicated the presence of a sequence homology with the CAP2 gene (GenbBank accession number: L40377). The sequence spanning the CAG repeat shown in Figure 15a was obtained by both cosmid sequencing and genome walker sequencing. The sequence was mapped between markers D18S68 and WI-3170 by STS content mapping.

A YAC 766-f-12 was fragmented using the (CAG)₇ or (CTG)₁₀ fragmentation vector. Again the (CTG)₁₀ vector did not reveal the presence of any CTG repeat. Analysis of twenty (CAG)₇ fragmented YACs showed the presence of two groups of fragments with the same size: five of approximatively 650kb and two of approximatively 50kb.

End Rescue was performed using ECORI on four of the fragmented YACs of 650kb. Sequencing confirmed that they all shared identical 3' terminals, characterised by the sequence shown in italics in Figure 16a. A Blast Search showed homology of this sequence with the Alu repeat sequence family. The sequence spanning the CAG repeat shown in Figure 16a was obtained by cosmid sequencing. The sequence was mapped between markers WI-2620 and WI-4211 by STS content mapping on the YAC contig map. End Rescue was also performed on the two fragments of 50kb. Sequencing revealed the sequence shown in italics in figure 17a. A Blast Search revealed no sequence homology with any known sequence. Cosmid sequencing allowed to identify the complete sequence spanning the CAG repeats, shown in figure 17a. The sequence was mapped between markers D18S968 and D18S875 by STS content mapping on the YAC contig map.

A YAC 907-e-1 clone was transformed with the (CAG)₇ or (CTG) ₁₀ fragmentation vector. The (CAG) ₇ vector did not reveal the presence of any CAG repeat. Analysis of twenty-six (CTG)₁₀ fragmented YACs revealed that twenty-one of them had the same size of approximatively 900kb. End Rescue was performed with KpnI on three fragmented YACS of this size. Sequencing revealed the nucleotide sequence shown in italics in Figure 18a. A Blast Search indicated the presence of an homology of this sequence with the GCT3GOI marker (GenBank accession number: G09484). The sequence spanning the CTG repeat was obtained from the GenBank Database. The sequence was mapped between markers 10R and WI-528.

## Claims

1. A method of determining the susceptibility of an individual to a bipolar disorder which method comprises analysing a sample of DNA obtained from that individual for the presence of a DNA polymorphism associated with a bipolar disorder in a region of human chromosome 18q disposed between polymorphic markers D18S68 and D18S979.

2. A method as in claim 1 wherein said DNA polymorphism is a trinucleotide repeat expansion.

3. A method as in claim 2 wherein said trinucleotide repeat expansion is comprised in a sequence of nucleotides that differ from the sequence of nucleotide shown in any one of Figures 15a, 16a, 17a or 18a only in said trinucleotide repeat expansion.

4. A method as in claim 2 or 3 which comprises the steps of:
a) obtaining a DNA sample from said individual;
b) providing primers suitable for the amplification of a nucleotide sequence comprised in the sequence shown in any one of Figures 15a, 16a, 17a or 18a said primers flanking the trinucleotide repeats comprised in said sequence;
c) applying said primers to the said DNA sample and carrying out an amplification reaction;
d) carrying out the same amplification reaction on a DNA sample from a control individual; and
e) comparing the results of the amplification reaction for the said individual and for the said control individual;
wherein the presence of an amplified fragment from said individual which is bigger in size from that of said control individual is an indication of the presence of a susceptibility to a bipolar disorder of said individual.

5. A method as in claim 4 wherein said nucleotide sequence to be amplified is comprised in the sequence shown in Figure 15a and said primers have the sequences shown in Figure 15b.

6. A method as in claim 4 wherein said nucleotide sequence to be amplified is comprised in the sequence shown in Figure 16a and said primers have the sequences shown in Figure 16b.

7. A method as in claim 4 wherein nucleotide sequence to be amplified is comprised in the sequence shown in Figure 17a and said primers have the sequences shown in Figure 17b.

8. A method as in claim 4 wherein said nucleotide sequence to be amplified is comprised in the sequence shown in Figure 18a and said primers have the sequences shown in Figure 18b.

9. Use of a region of human chromosome 18q disposed between polymorphic markers D18S68 and D18S979 or a fragment thereof for identifying at least one human gene, including mutated or polymorphic variants thereof, which contributes to the manifestation of bipolar disorder.

10. The use as claimed in claim 9 wherein said portion of human chromosome 18q is present in a YAC clone.

11. The use as claimed in claim 10 wherein said YAC clone is a clone from the CEPH Mega-YAC selected from 961_h_9, 942_c_3, 766_f_12, 731_c_7, 907_e_1, 752_g_8 or 717_d_3.

12. The use as claimed in claim 10 wherein said YAC clone is 961_h_9, 766_f_12 or 907_e_1.

13. The use as claimed in any of claims 9 to 12 wherein said bipolar disorder is selected from the Diagnostic and Statistical Manual of Mental Disorders, version 4 (DSM-IV) taxonomy and includes mood disorders (296.XX, 300.4, 311, 301, 13, 295.70), schizophrenia and related disorders (295, 297.1, 298.9, 297.3, 298.9), anxiety disorders (300.XX, 309.81, 308.3), adjustment disorders (309, XX) and personality disorders (codes 301.XX).

14. Use according to any one of claims 10 to 12 wherein the at least one human gene, including mutated or polymorphic variants thereof, which contributes to the manifestation of bipolar disorder is identified by a method comprising the steps of:
(a) subcloning a YAC clone according to any one of claims 10 to 12 into exon trap vectors;
(b) transfecting mammalian cells with an exon trap vector as prepared in step (a);
(c) culturing said mammalian cells in an appropriate medium;
(d) isolating RNA transcripts expressed from an SV40 promoter;
(e) preparing cDNA from said RNA transcripts;
(f) identifying splicing events involving exons of the DNA subcloned into said exon trap vector in accordance with step (a) to elucidate positions of coding regions in said subcloned DNA;
(g) detecting differences between said coding regions and equivalent regions in the DNA or an individual afflicted with said bipolar disorder; and
(h) identifying said gene or mutated or polymorphic variants thereof which contributes to the manifestation of bipolar disorder.

15. Use according to any one of claims 10 to 12 wherein the at least one human gene, including mutated or polymorphic variants thereof, which contributes to the manifestation of bipolar disorder is identified by a method comprising the steps of:
(a) subcloning a YAC clone according to any one of claims 10 to 12 into a cosmid, BAC, PAC or other vector;
(b) using the nucleotide sequences shown in any one of Figures 1 to 11 or any other known sequence tagged sequence from the YAC contig shown in Figure 14 or part thereof consisting of not less than 14 contiguous bases or the complement thereof, to defect overlaps amongst the subclones and construct a map thereof;
(c) identifying the position of genes within the subcloned DNA by one or more of CpG island identification, zoo-blotting, hybridization of said subcloned DNA to a cDNA library or a Northern blot of mRNA from a panel of culture cell lines;
(d) detecting differences between said genes and equivalent regions of the DNA of an individual afflicted with a bipolar disorder; and
(e) identifying said gene which, if defective, contributes to the manifestation of bipolar disorder.

## Patentansprüche

1. Verfahren zum Bestimmen der Anfälligkeit eines Individuums für eine bipolare Störung, worin das Verfahren Analysieren einer von dem Individuum erhaltenen DNA-Probe bezüglich des Vorliegens eines DNA-Polymorphismus, welcher mit einer bipolaren Störung in Zusammenhang steht, in einer Region des humanen Chromosoms 18q, welche zwischen den polymorphen Markern D18S68 und D18S979 angeordnet ist, umfasst.

2. Verfahren nach Anspruch 1, worin der DNA-Polymorphismus eine Trinukleotid-Repeat-Expansion ist.

3. Verfahren nach Anspruch 2, worin die Trinukleotid-Repeat-Expansion in einer Nukleotidsequenz enthalten ist, welche sich von der in einer der Figuren 15a, 16a, 17a oder 18a gezeigten Nukleotidsequenzen nur in der Trinukleotid-Repeat-Expansion unterscheidet.

4. Verfahren nach Anspruch 2 oder 3, welches die Schritte umfasst:
a) Erhalten einer DNA-Probe von dem Individuum;
b) Bereitstellen von Primern, welche für die Amplifikation einer Nukleotidsequenz geeignet sind, welche in einer der Figuren 15a, 16a, 17a oder 18a gezeigten Nukleotidsequenzen enthalten ist, worin die Primer die in der Sequenz gezeigten Trinukleotid-Repeats flankieren;
c) Einsetzen der Primer bei der DNA-Probe und Durchführen einer Amplifikationsreaktion;
d) Durchführen der gleichen Amplifikationsreaktion mit einer DNA-Probe eines Kontrollindividuums; und
e) Vergleichen der Ergebnisse der Amplifikationsreaktion des Individuums und der des Kontrollindividuums;
worin das Vorliegen eines amplifizierten Fragments von dem Individuum, welches größer ist als das des Kontrollindividuums, ein Anzeichen für das Vorliegen einer Anfälligkeit für eine bipolare Störung des Individuums ist.

5. Verfahren nach Anspruch 4, worin die zu amplifizierende Nukleotidsequenz in der in Figur 15a gezeigten Sequenz enthalten ist, und worin die Primer die in Figur 15b gezeigten Sequenzen aufweisen.

6. Verfahren nach Anspruch 4, worin die zu amplifizierende Nukleotidsequenz in der in Figur 16a gezeigten Sequenz enthalten ist, und worin die Primer die in Figur 16b gezeigten Sequenzen aufweisen.

7. Verfahren nach Anspruch 4, worin die zu amplifizierende Nukleotidsequenz in der in Figur 17a gezeigten Sequenz enthalten ist, und worin die Primer die in Figur 17b gezeigten Sequenzen aufweisen.

8. Verfahren nach Anspruch 4, worin die zu amplifizierende Nukleotidsequenz in der in Figur 18a gezeigten Sequenz enthalten ist, und worin die Primer die in Figur 18b gezeigten Sequenzen aufweisen.

9. Verwendung einer Region des humanen Chromosoms 18q, welche zwischen den polymorphen Marken D18S68 und D18S979 angeordnet ist, oder eines Fragments davon zum Identifizieren zumindest eines humanen Gens, einschließlich mutierter oder polymorpher Varianten davon, welches zur Manifestation einer bipolaren Störung beiträgt.

10. Verwendung nach Anspruch 9, worin der Anteil des humanen Chromosoms 18q in einem YAC-Klon vorliegt.

11. Verwendung nach Anspruch 10, worin der YAC-Klon ein Klon des CEPH Mega-YACs ist, welcher ausgewählt ist aus 961_h_9, 942_c_3, 766_f_12, 731_c_7, 907_e_1, 752_g_8 oder 717_d_3.

12. Verwendung nach Anspruch 10, worin der YAC-Klon 961_h_9, 766_f_12 oder 907_e_1 ist.

13. Verwendung nach einem der Ansprüche 9 bis 12, worin die bipolare Störung ausgewählt ist aus der Taxonomie des diagnostischen und statistischen Handbuch psychischer Erkrankungen "(Diagnostic and Statistical Manual of Mental Disorders"), Version 4 (DSM-IV), und Gemütskrankheiten (296.XX, 300.4, 311, 301, 13, 295.70), Schizophrenie und verwandte Erkrankungen (295, 297.1, 298.9, 297.3, 298.9), Angsterkrankungen (300.XX, 309.81, 308.3), Anpassungsstörungen (309, XX) und Persönlichkeitsstörungen (Code 301.XX) umfasst.

14. Verwendung nach einem der Ansprüche 10 bis 12, worin das zumindest eine humane Gen, einschließlich mutierter oder polymorpher Varianten davon, welches zur Manifestation einer bipolaren Störung beiträgt, durch ein Verfahren identifiziert wird, welches die Schritte umfasst:
(a) Subklonieren eines YAC-Klons, wie in einem der Ansprüche 10 bis 12 beschrieben, in Exon-Trap-Vektoren;
(b) Transfizieren von Säugerzellen mit einem in Schritt (a) hergestellten Exon-Trap-Vektor;
(c) Kultivieren der Säugerzellen in einem geeigneten Medium;
(d) Isolieren von RNA-Transkripten, welche von einem SV40-Promotor exprimiert werden;
(e) Herstellen von cDNA von solchen RNA-Transkripten;
(f) Identifizieren von Spleiß-Ereignissen, welche die Exons der in den gemäß Schritt (a) in den Exon-Trap-Vektor subklonierten DNA betreffen, um die Positionen der codierenden Regionen in der subklonierten DNA zu identifizieren;
(g) Nachweisen von Unterschieden zwischen den codierenden Regionen und äquivalenten Regionen in der DNA eines Individuums, welches unter der bipolaren Störung leidet; und
(h) Identifizieren des Gens oder von mutierten oder polymorphen Varianten davon, welches bzw. welche zu der Manifestation der bipolaren Störung beiträgt bzw. beitragen.

15. Verwendung nach einem der Ansprüche 10 bis 12, worin das zumindest eine humane Gen, einschließlich mutierter oder polymorpher Varianten davon, welches zu der Manifestation einer bipolaren Störung beiträgt, durch ein Verfahren identifiziert wird, welches die Schritte umfasst:
(a) Subklonieren eines YAC-Klons, wie in einem der Ansprüche 10 bis 12 beschrieben, in einen Cosmid-, BAC-, PAC-oder einen anderen Vektor;
(b) Verwenden der in einer der Figuren 1 bis 11 gezeigten Nukleotidsequenzen oder einer beliebigen anderen bekannten, mit einem Tag versehenen Sequenz aus dem in Figur 14 gezeigten YAC-Contig, oder eines Teils davon, welcher aus nicht weniger als 14 benachbarten Basen besteht, oder dem Komplement davon, um Überlappungen zwischen den Subklonen nachzuweisen und eine Karte davon zu erstellen;
(c) Identifizieren der Positionen der Gene in der subklonierten DNA durch eines oder mehrere von CpG-Insel-Identifizierung, Zoo-Blotting, Hybridisierung der subklonierten DNA mit einer cDNA-Bibliothek oder einen Northern-Blot von mRNA aus einem Panel von Kulturzelllinien;
(d) Nachweisen von Unterschieden zwischen den Genen und äquivalenten Regionen der DNA von einem Individuum, welches unter einer bipolaren Störung leidet; und
(e) Identifizieren des Gens, welches, wenn es fehlerhaft ist, zu der Manifestation einer bipolaren Störung beiträgt.

## Revendications

1. Procédé de détermination de la sensibilité d'un individu à un trouble bipolaire, lequel procédé comprend l'analyse d'un échantillon d'ADN obtenu à partir de cet individu visant à déterminer la présence d'un polymorphisme d'ADN associé à un trouble bipolaire dans une région du chromosome humain 18q disposée entre les marqueurs polymorphiques D18S68 et D18S979.

2. Procédé selon la revendication 1, dans lequel ledit polymorphisme d'ADN est une expansion de répétitions de trinucléotides.

3. Procédé selon la revendication 2, dans lequel ladite expansion de répétitions de trinucléotides est comprise dans une séquence de nucléotides qui diffère de la séquence de nucléotides représentée sur l'une quelconque des figures 15a, 16a, 17a ou 18a uniquement par ladite expansion de répétitions de trinucléotides.

4. Procédé selon la revendication 2 ou 3 qui comprend les étapes consistant à :
a) obtenir un échantillon d'ADN à partir dudit individu ;
b) fournir des amorces appropriées pour l'amplification d'une séquence de nucléotides comprise dans la séquence représentée sur l'une quelconque des figures 15a, 16a, 17a ou 18a, lesdites amorces flanquant les répétitions de trinucléotides comprises dans ladite séquence ;
c) appliquer lesdites amorces au dit échantillon d'ADN et mettre en oeuvre une réaction d'amplification ;
d) mettre en oeuvre la même réaction d'amplification sur un échantillon d'ADN d'un individu témoin ; et
e) comparer les résultats de la réaction d'amplification pour ledit individu et pour ledit individu témoin ;
où la présence d'un fragment amplifié dudit individu qui est de taille plus importante que celui dudit individu témoin est un indice de la présence d'une sensibilité à un trouble bipolaire dudit individu.

5. Procédé selon la revendication 4, dans lequel ladite séquence de nucléotides à amplifier est comprise dans la séquence représentée sur la figure 15a et lesdites amorces ont les séquences représentées sur la figure 15b.

6. Procédé selon la revendication 4, dans lequel ladite séquence de nucléotides à amplifier est comprise dans la séquence représentée sur la figure 16a et lesdites amorces ont les séquences représentées sur la figure 16b.

7. Procédé selon la revendication 4, dans lequel la séquence de nucléotides à amplifier est comprise dans la séquence représentée sur la figure 17a et lesdites amorces ont les séquences représentées sur la figure 17b.

8. Procédé selon la revendication 4, dans lequel ladite séquence de nucléotides à amplifier est comprise dans la séquence représentée sur la figure 18a et lesdites amorces ont les séquences représentées sur la figure 18b.

9. Utilisation d'une région du chromosome humain 18q placée entre les marqueurs polymorphiques D18S68 et D18S979 ou d'un fragment de celle-ci afin d'identifier au moins un gène humain, comprenant les variantes polymorphiques ou ayant muté de celui-ci, qui contribue à la manifestation du trouble bipolaire.

10. Utilisation selon la revendication 9, dans laquelle ladite partie du chromosome humain 18q est présente dans un clone YAC.

11. Utilisation selon la revendication 10, dans laquelle ledit clone YAC est un clone du Méga-YAC CEPH sélectionné à partir de 961_h_9, 942_c_3, 766_f_12, 731_c_7, 907_e_1, 752_g_8 ou 717_d_3.

12. Utilisation selon la revendication 10, dans laquelle ledit clone YAC est 961_h_9, 766_f_12 ou 907_e_1.

13. Utilisation selon l'une quelconque des revendications 9 à 12, dans laquelle ledit trouble bipolaire est sélectionné à partir de la taxonomie de la 4ème édition du Manuel Diagnostique et Statistique des troubles mentaux (DSM-IV) et comprend les troubles de l'humeur (296.XX, 300.4, 311, 301, 13, 295.70), la schizophrénie et les troubles associés (295, 297.1, 298.9, 297.3, 298.9), les troubles de l'anxiété (300.XX, 309.81, 308.3), les troubles de l'adaptation (309, XX) et les troubles de la personnalité (codes 301.XX).

14. Utilisation selon l'une quelconque des revendications 10 à 12, dans laquelle le au moins un gène humain, y compris les variantes polymorphiques ou ayant muté de celui-ci, qui contribue à la manifestation du trouble bipolaire est identifié par un procédé comprenant les étapes consistant à :
(a) sous-cloner un clone YAC selon l'une quelconque des revendications 10 à 12 dans des vecteurs de piégeage d'exon ;
(b) transfecter des cellules de mammifère avec un vecteur de piégeage d'exon tel que préparé dans l'étape (a) ;
(c) cultiver lesdites cellules de mammifère dans un milieu approprié ;
(d) isoler les produits de transcription d'ARN exprimés à partir d'un promoteur SV40 ;
(e) préparer l'ADNc à partir des produits de transcription d'ARN ;
(f) identifier les événements d'épissage impliquant des exons de l'ADN sous-cloné dans ledit vecteur de piégeage d'exon selon l'étape (a) afin de déterminer les positions des régions codantes dans ledit ADN sous-cloné ;
(g) détecter les différences entres lesdites régions codantes et les régions équivalentes dans l'ADN ou un individu atteint dudit trouble bipolaire ; et
(h) identifier ledit gène, ou les variantes polymorphiques ou ayant muté de celui-ci, qui contribue à la manifestation du trouble bipolaire.

15. Utilisation selon l'une quelconque des revendications 10 à 12, dans laquelle le au moins un gène humain, y compris les variantes polymorphiques ou ayant muté de celui-ci, qui contribue à la manifestation du trouble bipolaire est identifié par un procédé comprenant les étapes consistant à :
(a) sous-cloner un clone YAC selon l'une quelconque des revendications 10 à 12 dans un cosmide, un BAC, un PAC ou un autre vecteur;
(b) utiliser les séquences de nucléotides représentées sur l'une quelconque des figures 1 à 11 ou une quelconque autre séquence connue, marquée par une séquence, du contig YAC représenté sur la figure 14 ou une partie de celle-ci constituée de pas moins de 14 bases contiguës ou du complément de celle-ci, afin de détecter les chevauchements parmi les sous-clones et construire une carte de ceux-ci ;
(c) identifier la position des gènes dans l'ADN sous-cloné grâce à une ou plusieurs techniques parmi l'identification d'îlots CpG, le « zoo-blotting », l'hybridation dudit ADN sous-cloné à une bibliothèque d'ADNc ou à un transfert d'ARNm à partir d'un panel de lignées de cellules de culture ;
(d) détecter les différences entre lesdits gènes et les régions équivalentes de l'ADN d'un individu atteint d'un trouble bipolaire ; et
(e) identifier ledit gène qui, s'il est déficient, contribue à la manifestation du trouble bipolaire.
